(19) Europäisches Patentamt
European Patent Office
Office européen des brevets

(11) Numéro de publication: **0 088 023**
**B1**

(12) # FASCICULE DE BREVET EUROPÉEN

(45) Date de publication du fascicule du brevet: **14.01.87**

(21) Numéro de dépôt: **83400400.4**

(22) Date de dépôt: **25.02.83**

(51) Int. Cl.⁴: **C 07 D 491/14**, C 07 D 407/12, A 61 K 31/44, A 61 K 7/42 // (C07D491/14, 311:00, 307:00, 221:00)

(54) **Pyrido(3',4':4,5) furo(3,2-g) coumarines ou pyrido(3,4-h) psoralènes, obtention, applications en cosmétologie et à titre de médicaments, et compositions cosmétiques et pharmaceutiques les contenant.**

(30) Priorité: **25.02.82 FR 8203158**

(43) Date de publication de la demande: **07.09.83 Bulletin 83/36**

(45) Mention de la délivrance du brevet: **14.01.87 Bulletin 87/3**

(84) Etats contractants désignés: **AT BE CH DE GB IT LI LU NL SE**

(56) Documents cité: **FR-A-2 405 067**

(73) Titulaire: **INSTITUT NATIONAL DE LA SANTE ET DE LA RECHERCHE MEDICALE (INSERM), 101, rue de Tolbiac, F-75654 Paris Cedex 13 (FR)**

(72) Inventeur: **Bisagni, Emile, 16, rue Bossuet, F-91400 Orsay (FR)**
Inventeur: **Moron, Jacqueline, 6 Hameau de l'Yvette Bât.E, F-91190 Gif- sur- Yvette (FR)**
Inventeur: **Averbeck, Dietrich, 2bis av. Général de Gaulle, F-94240 L'Hay- les- Roses (FR)**
Inventeur: **Dubertret, Louis, 31, avenue du Président Coty, F-75014 Paris (FR)**

(74) Mandataire: **Phélip, Bruno, c/o Cabinet Harlé & Phélip 21, rue de la Rochefoucauld, F-75009 Paris (FR)**

**EP 0 088 023 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention concerne le domaine des dérivés du psoralène. Elle a plus particulièrement pour objet des dérivés mono-fonctionnels du psoralène, à savoir des pyrido[3',4':4,5]furo[3,2-g]coumarines ou pyrido-[3,4-h]psoralènes. L'invention a également pour objet un procédé d'obtention de tels composés ainsi que des intermédiaires qui apparaissent dans le procédé. L'invention concerne aussi l'application desdits composés en cosmétologie, notamment pour stimuler la pigmentation de la peau. En plus, elle concerne les composés mentionnés pour l'application à titre de médicaments, en particulier pour le traitement des affections cutanées. L'invention est aussi relative à des compositions cosmétiques et pharmaceutiques contenant, à titre d'agent actif, lesdits composés.

La photochimiothérapie, qui repose sur l'activation par les ultraviolets A, dans la peau humaine, de molécules photo-actives de la famille des furocoumarines, les psoralènes, se développe rapidement en dermatologie. Il s'agit en effet d'une technique thérapeutique particulièrement efficace et commode dans le traitement des dermatoses inflammatoires chroniques bénignes, comme le psoriasis qui touche 2 à 3% de la population mondiale, et dans le traitement de dermatoses malignes, comme le mycosis fongoïde, lymphome cutané malin, rare mais d'évolution particulièrement sévère. Cette technique thérapeutique est également employée avec un certain succès dans le traitement de nombreuses maladies inflammatoires chroniques de la peau, comme l'eczéma atypique, le lichen plan, le parapsoriasis en goutte, le prurit des hémodialysés, ainsi que dans les photodermatoses et dans les maladies dépigmentantes comme le vitiligo.

A titre de document illustrant l'art antérieur, on peut citer la demande de brevet français n° 78 24 754 (publication n° 2 405 067) déposée le 25 août 1978 au nom de l'AGENCE NATIONALE DE VALORISATION DE LA RECHERCHE (ANVAR), ayant pour titre "Compositions pharmaceutiques contenant des dérivés mono-fonctionnels du psoralène pour le traitement des affections cutanées".

D'une manière résumée, on sait que, pour traiter des affections cutanées, telles que le psoriasis, on administre certaines furocoumarines ou psoralènes en association avec la lumière ultraviolette proche (UVA). Ce traitement de photothérapie est connu sous le nom de PUVA thérapie.

Les travaux les plus récents ont montré que certains psoralènes bifonctionnels, tels que le 8-méthoxy psoralène (en abréviation 8-MOP) et le 5-méthoxy psoralène (en abréviation 5-MOP) dans les conditions du traitement c'est-à-dire en présence de lumière ultraviolette proche (UVA), possèdent des effets cancérigènes chez la souris. Au contraire, le 3-carbéthoxy psoralène (en abréviation 3-CPs) est un dérivé monofonctionnel du psoralène totalement inoffensif de ce point de vue. Il a été également démontré que le 3-Cps conservait une activité thérapeutique vis-à-vis des malades atteints de psoriasis.

Les psoralènes actuellement utilisés en thérapie, tels le 8-méthoxypsoralène ou le 5-méthoxypsoralène, sont des agents capables de réaliser des pontages dans l'-ADN, pontages biologiquement difficiles à réparer et capables d'introduire des erreurs dans la réplication génétique. Cette propriété explique sans doute leur haute activité mutagène et leur propriété carcinogène chez l'animal. Il s'agit donc de médicaments efficaces mais peut-être non dénués de risques en utilisation chronique chez l'homme. C'est pourquoi, la photochimiothérapie est interdite chez les enfants jusqu'à l'âge de 18 à 20 ans et l'on essaye de l'utiliser le moins possible avant la cinquantaine.

En cosmétologie, on connaît aussi des produits qui stimulent la pigmentation et sont constitués de dérivés du psoralène, tels que le 5-méthoxy psoralène ou 5-MOP. Ils trouvent application dans des compositions cosmétiques pour le brunissage de la peau et/ou la protection de celle-ci contre le soleil. L'inconvénient de tels produits est d'être mutagènes.

La présente invention a pour objet de nouveaux dérivés monofonctionnels du psoralène qui présentent des propriétés permettant d'améliorer la PUVA thérapie et la stimulation de la pigmentation.

Ces composés sont utiles à titre de médicaments, notamment dans le domaine du traitement des affections cutanées et plus spécialement pour le traitement des dermatoses inflammatoires bénignes et malignes, à savoir le psoriasis, le mycosis fongoïde, les eczémas constitutionnels et de contact, les parapsoriasis en plaques et en gouttes, les pelades, les prurigos, les lichens plans, les urticaires pigmentaires ainsi que les troubles de la pigmentation et les photodermatoses.

Les composés de l'invention qui possèdent l'aptitude de stimuler la pigmentation de la peau, sont également utiles dans le domaine cosmétique.

L'invention concerne des pyrido[3,4-h]psoralènes ou pyrido[3',4':4,5]furo[3,2-g]coumarines répondant à la formule I

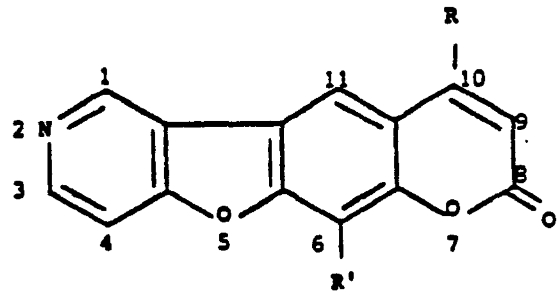

(I)

formule dans laquelle R est un atome d'hydrogène ou un groupe alkyle inférieur e $C_1$-$C_4$ et R' est un groupe méthyle ou méthoxy. De préférence, R est un groupe méthyle ou un atome d'hydrogène.

L'invention concerne également des procédés pour l'obtention des nouveaux dérivés de formule I.

Dans un premier procédé de préparation, on part d'une aminooxy-7coumarine substituée en position 8 par un groupe méthyle ou méthoxy et portant en position 4 un atome d'hydrogène ou un radical alkyle inférieur, de préférence un radical méthyle. Un tel produit de départ peut être représenté par la formule II ci-après.

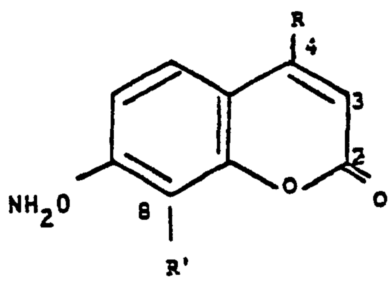

(II)

dans laquelle R et R' ont la signification mentionnée cidessus.

Le produit de départ de formule II peut être obtenu par la technique décrite par DEAN R. BENDER et al. dans Journal of Organic Chemistry, 4, 2176 (1979) "Psoralen Synthesis. Improvements in Furano Ring Formation. Application to the Synthesis of 4,5',8-trimethyl-psoralen". Dans son adaptation aux fins de la présente invention, cette technique est mise en oeuvre en préparant d'abord l'hydroxycoumarine appropriée en partant du phénol correspondant, selon une réaction connue sous le nom de réaction de VON PECHMANN. Il s'agit d'une réaction de condensation, connue de l'homme de l'art (voir Organic Reactions, T 7, p. 1).

La réaction de VON PECHMANN est utilisée pour la synthèse des coumarines et implique la condensation d'un β-cétoester sur un phénol en particulier un polyphénol ou un acétate d'un tel phénol. La réaction est réalisée en milieu acide. Selon la technique de BENDER et al., l'hydroxycoumarine obtenue après la réaction de VON PECHMANN est mise à réagir avec l'hydrure de sodium dans le diméthylformamide, puis avec la 0-2,4-dinitrophénylhydroxylamine. On obtient ainsi le produit de départ de formule II.

La première étape du procédé de l'invention consiste à faire réagir le produit (II) sur une 4-pipéridone de formule III

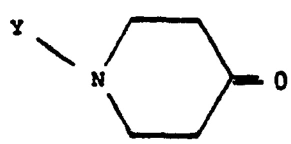

(III)

dans laquelle le substituant Y peut être un atome d'hydrogène, un radical méthyle ou un groupe benzyle. Cette réaction est réalisée avantageusement à la température ordinaire et sous pression normale en milieu alcoolique acidifié. Le milieu préféré est constitué d'éthanol additionné d'acide chlorhydrique. Dans ce cas le chlorhydrate obtenu précipite et peut être aisément isolé par les moyens usuels. On obtient un produit intermédiaire, qui est une oxime de formule IV

HCl, 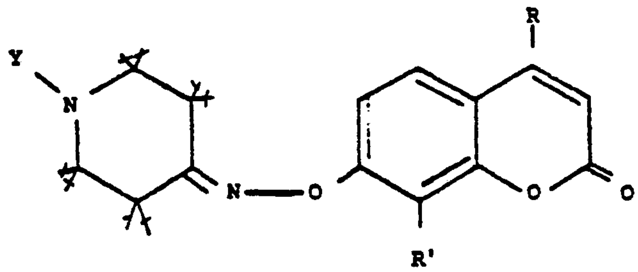 (IV)

dans laquelle R, R' et Y ont les significations indiquées ci-dessus.

La deuxième étape du procédé de l'invention consiste en une cyclisation de l'oxime IV. A cet effet, l'oxime IV est mise en solution dans un milieu acide fort, de préférence dans l'acide acétique additionné d'acide chlorhydrique, avantageusement sous forme de HCl gazeux sec. Le milieu le plus préférentiel est constitué d'acide acétique additionné de 6% environ d'HCl gazeux sec. La réaction est effectuée à chaud, à savoir entre 70 et 90°C environ, de préférence aux environs de 80°C.

Cette deuxième étape conduit à un produit intermédiaire de formule V

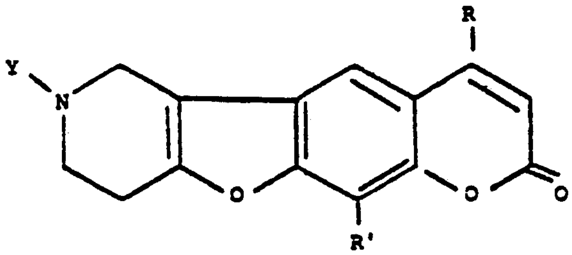 (V)

dans laquelle R, R' et Y ont la signification indiquée ci-dessus. Le produit V est un tétrahydro-1,2,3,4pyrido-[3,4-h]psoralène ou tétrahydro-1,2,3,4pyrido[3',4':4,5]-furo[3,2-g]coumarine. Avec un rendement de 60 à 75%, on obtient sous forme d'un précipité le chlorhydrate du composé V. Ce précipité peut donc être facilement isolé par les techniques usuelles. Des sous-produits de la réaction se trouvent dans les eaux-mères mais n'intéressent pas directement le procédé de l'invention.

La troisième et dernière étape du procédé de l'invention consiste en une réaction d'aromatisation en partant du produit intermédiaire V précité. Cette réaction est réalisée en milieu solvant en présence d'un agent de déshydrogénation ayant une forte activité, de préférence le palladium sur charbon, par exemple à 10%. Le solvant préféré est la décaline. On travaille à la température d'ébullition c'est-à-dire au reflux du solvant. Au lieu de décaline, on peut utiliser tout autre solvant capable de remplir sa fonction à la température de reflux et d'être inerte vis-à-vis des réactifs en présence. Un exemple d'un tel solvant est le diphényléther. Au cours de la réaction d'aromatisation le groupe Y protecteur de l'atome d'azote s'élimine et on obtient le dérivé désiré de psoralène de formule I.

Le procédé qui vient d'être décrit en détail cidessus convient pour la préparation de tous les nouveaux composés de formule I.

Selon l'invention, on a également mis au point un autre procédé qui, à titre de variante, peut être utilisé pour l'obtention des composés de formule I dans lesquels R est un atome d'hydrogène et R' un radical méthyle ou un radical méthoxy. Dans ce procédé on part de l'éther monométhylique de la méthyl-2- ou méthoxy-2 résorcine de formule VI

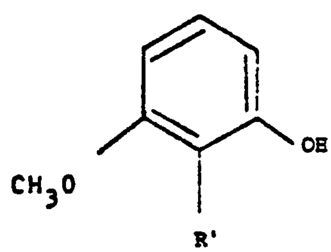 (VI)

dans laquelle R' = $CH_3$ ou $OCH_3$. Le composé de départ de formule VI avec R' = $CH_3$ est obtenu à partir de la méthyl-2 résorcine, produit disponible dans le commerce et le composé de départ de formule VI avec R' = $OCH_3$ est décrit dans Beilstein, T 6, p. 1081.

L'autre produit de départ est la nitro-3-chloro-4 pyridine de formule VII

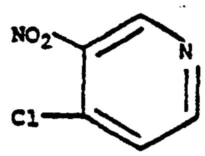

(VII)

Le composé de formule VII peut être préparé par des moyens connus par exemple comme décrit par R.R. Bishop et al, J. Chem. Soc. 1952, p. 437.

La première étape de cette variante de procédé selon l'invention consiste à faire réagir les composés VI et VII. En général on prépare d'abord le sel alcalin du phénol (VI) qu'on condense avec la nitro-3chloro-4 pyridine. On travaille dans un solvant, tel que le diméthylformamide, en chauffant en fin de réaction pour compléter celle-ci. On obtient ainsi un dérivé nitré intermédiaire répondant à la formule VIII

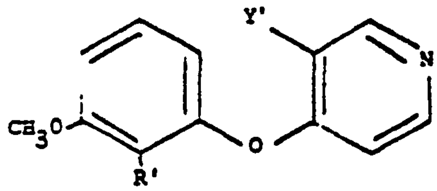

(VIII)

dans laquelle R' est $CH_3$ ou $OCH_3$ et Y' = $NO_2$.

On transforme le produit intermédiaire VIII dans lequel Y' = $NO_2$ en l'amine correspondante dans laquelle Y' = $NH_2$ par réduction catalytique, c'est-à-dire par traitement à l'hydrogène en présence de nickel Raney ou de charbon palladié. L'amine de formule VIII dans laquelle Y' = $NH_2$ est ensuite diazotée en milieu chlorhydrique par le nitrite de sodium pour fournir un produit diazoté qui n'a pas besoin d'être isolé et qui est traité immédiatement. A cet effet, le produit diazoté correspondant à la formule VIII dans laquelle Y' représente le radical $N_2^+Cl^-$ est cyclisé en présence de sel cuivrique (le chlorure cuivrique par exemple) selon la réaction de Meerwein (Organic Reactions, T 11, p. 189).

On obtient ainsi à titre de produit intermédiaire le composé de formule IX

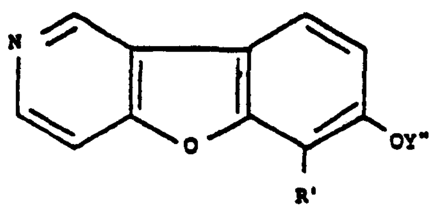

(IX)

dans laquelle Y'' représente $CH_3$ et R' a la signification indiquée ci-dessus à savoir $CH_3$ ou $OCH_3$. Ce produit est alors soumis à une réaction de déméthylation, en particulier avec du chlorure de pyridinium bouillant, pour fournir le composé correspondant de formule IX dans lequel Y'' est un atome d'hydrogène. On obtient ainsi un hydroxypyridobenzofuranne de formule IX dans lequel Y'' = H. Ce dernier composé est alors soumis à une réaction de formylation à l'aide d'hexaméthylènetétramine dans l'acide acétique, suivie d'hydrolyse. On obtient ainsi le produit intermédiaire de formule X

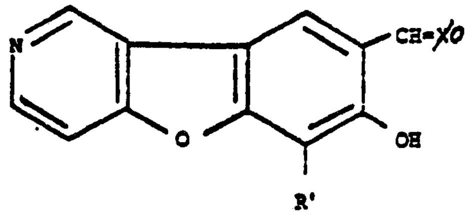

(X)

dans laquelle R' représente un radical méthyle ou méthoxy.

L'aldéhyde de formule X est alors condensé avec le diéthylmalonate dans un alcool tel que l'éthanol en présence d'une base, de préférence la pipéridine.

On obtient ainsi un carbéthoxypyrido-psoralène de formule XI

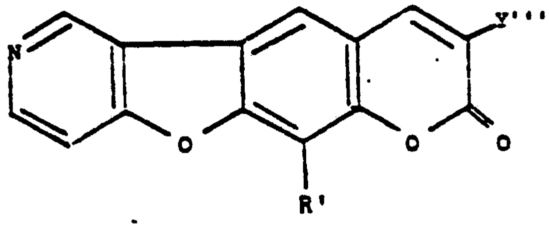

(XI)

dans laquelle Y''' représente le radical $COOC_2H_5$ et R' = $CH_3$ ou bien $OCH_3$. Ce produit est alors hydrolysé, ce qui fournit l'acide correspondant de formule XI dans laquelle Y''' = COOH. L'hydrolyse est réalisée en milieu acide.

Une réaction classique de décarboxylation permet d'obtenir le pyridopsoralène de formule I dans laquelle R = H et R' = $CH_3$ ou bien $OCH_3$.

Cette variante du procédé de préparation selon l'invention est illustrée par le schéma réactionnel ciaprès, dans le cas particulier où R = H et R' = $CH_3$.

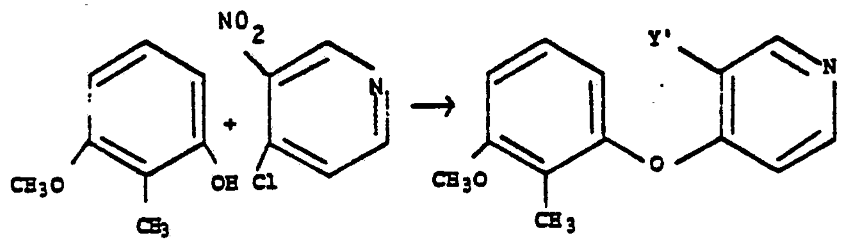

Composé 6 Composé 7 Composés 8a $Y'=NO_2$

8b $Y'=NH_2$

8c $Y'=N_2^+Cl^-$

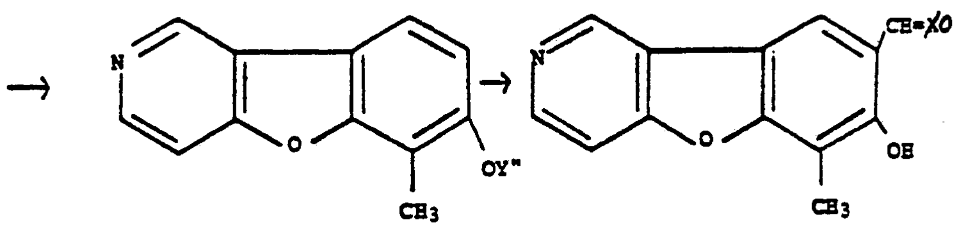

composés 9a $Y''=CH_3$

9b $Y''=H$

Composé 10a ~~(X=O)~~

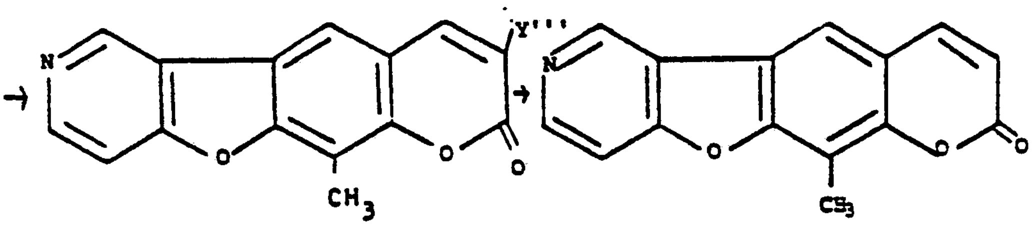

Composés 11a $Y'''=COOC_2H_5$

11b $Y'''=COOH$

Les composés particuliers du schéma réactionnel ci-dessus, qui portent les références 6, 7, 8a, 8b, 8c, 9a, 9b, 10a, 11a et 11b sont caractérisés dans les exemples qui suivent.

Les nouveaux composés selon l'invention, de formule I sont utiles à titre de médicaments. Certains intermédiaires, tels qu'utilisés dans le procédé de l'invention, présentent aussi une activité biologique. C'est le cas notamment des produits de formule V. Les nouveaux médicaments contenant à titre d'agent actif au moins l'un des composés selon l'invention sont applicables pour le traitement de diverses affections cutanées, et plus spécialement pour le traitement des dermatoses inflammatoires, bénignes et malignes, à savoir le psoriasis, le mycosis fongoïde, les eczémas constitutionnels et de contact, les parapsoriasis en plaques et en gouttes, les pelades, les prurigos, les lichens plans, les urticaires pigmentaires ainsi que les troubles de la pigmentation et les photodermatoses.

Les composés de l'invention qui possèdent l'aptitude de stimuler la pigmentation de la peau, sont également utiles dans le domaine cosmétique.

Le traitement des affections cutanées avec les composés selon la présente invention consiste à administrer par voie orale ou topique, une quantité efficace d'un composé et à soumettre le patient à de la lumière dans le proche UV (UVA).

Il a été constaté à présent que, pour l'application topique des pommades ou des solutions contenant environ 0,1% à environ 2% (en poids) du composé thérapeutiquement efficace peuvent être utilisées. Des concentrations d'environ 0,5% à 2% sont préférées.

Comme excipients pour les solutions ou les pommades selon l'invention, on peut utiliser tous les excipients couramment employés qui sont bien connus de l'homme de l'art. Des exemples de tels excipients sont cités par Schäefer et al. dans Archiv. of Dermatology (1976), ce document étant mentionné ici à titre de référence.

Il est bien entendu possible également d'incorporer les composés de la présente invention dans d'autres compositions, solutions ou pommades ou d'ajouter des parfums, des colorants, des filtres solaires, ou des agents de conservation, ainsi que tous autres composés couramment utilisés. Il est également possible de combiner deux ou plusieurs agents photosensibilisateurs ou photoprotecteurs.

Pour l'administration orale, on préfère en particulier des quantités comprises entre 0,5 et 2 mg/kg.

Ainsi qu'il est mentionné plus haut, le traitement des affections cutanées avec les composés selon l'invention comprend l'irradiation par de la lumière dans le proche ultraviolet (UVA) avec un spectre comprenant essentiellement des longueurs d'onde comprises entre 320 et 380 nm. Ainsi qu'on l'expliquera plus loin, la dose d'irradiation à chaque séance peut être d'emblée de 5 $J/cm^2$ à 10 $J/cm^2$. Néanmoins, des doses allant jusqu'à 20 $J/cm^2$ peuvent être utilisées pendant une courte période de temps.

Les composés de l'invention trouvent également application en cosmétologie, en raison de leur propriété de stimuler la pigmentation de la peau. L'invention a donc pour objet des compositions cosmétiques contenant une quantité d'au moins un composé selon l'invention efficace pour réaliser la pigmentation de la peau, en association avec un véhicule convenant à l'application externe. Les véhicules de telles compositions sont bien connus de l'homme de l'art et n'ont pas besoin d'être décrits plus en détails. Pour les composés de l'invention on peut utiliser des véhicules semblables à ceux déjà proposés pour les compositions cosmétiques contenant du 5-MOP. Les compositions cosmétiques de l'invention se présentent sous forme de crème, lait, huile, aérosols (sprays) et tous autres produits à usage externe. Les composés de l'invention peuvent, au sein de telles compositions, être associés ou non à des filtres ou écrans solaires. La quantité de composé actif à mettre en oeuvre dans les compositions cosmétiques de l'invention n'est pas critique et peut varier selon la destination de celles-ci. En général des quantités de 0,01 à 0,5% en poids par rapport à la composition sont convenables.

Les nouveaux composés de l'invention présentent un ensemble de propriétés qui les rendent particulièrement intéressants pour le traitement des affections cutanées, à savoir:

. une photoréactivité appréciable avec les acides nucléiques, produisant un effet antiprolifératif;

. une absence d'induction de pontages dans l'ADN;

. une induction dans l'ADN de mono-additions uniquement ayant pour conséquence l'arrêt des synthèses d'ADN et d'ARN;

. de faibles effets phototoxiques donc provoquant peu ou pas d'érythème;

. un faible pouvoir mutagène, ce qui contraste avec les furocoumarines bi-fonctionnelles actuellement employées en PUVA thérapie qui ont un pouvoir mutagène important.

Les exemples ci-après qui sont donnés à titre purement illustratif illustrent la préparation et les propriétés des nouveaux composés de l'invention.

EXEMPLE 1

Etape a) Nitro-3 (méthyl-2' méthoxy-3' phénoxy-1')-4 pyridine, (composé 8a, formule VIII, $R' = CH_3$, $Y = NO_2$)

Le composé 6, à savoir le méthyl-2-méthoxy-3-phénol (4,35 g, 31,5 mmoles) est ajouté à une solution de potasse (1,76 g) dans l'éthanol (40 ml) et la solution résultante est évaporée à sec. Le résidu est dissous dans la diméthylformamide (DMF) (50 ml). La nitro-3 chloro-4 pyridine 7 (5g, 31,5 mmoles) en solution dans la DMF (50 ml) est ajoutée en une seule fois. Le mélange est agité à la température ambiante pendant 1 h puis chauffé à 60°C pendant 1 h supplémentaire et évaporé à sec sous pression réduite. Le résidu, repris dans l'eau, est

extrait au chloroforme, lavé à l'eau, et la phase organique évaporée fournit un résidu qui recristallise dans l'alcool en présence de charbon animal pour donner 5g (61%) de cristaux jaunes, F = 115-117°C.

Analyse % calculé pour $C_{13}H_{12}N_2O_4$: C 60,00 H 4,6 N 10,7
Trouvé: 59,8 4,6 10,7

Etape b) Amino-3 (méthyl-2' méthoxy-3' phénoxy-1')-4 pyridine (composé 8b, formule VIII, R' = $CH_3$, Y = $NH_2$)

Le dérivé nitré 8a obtenu à l'exemple 1 (5g, 19,2 mmoles) est dissous dans l'éthanol absolu (100 ml) et hydrogéné par agitation sous atmosphère d'hydrogène en présence de nickel de Raney (5g) jusqu'à absorption de la quantité théorique d'hydrogène. Le catalyseur est filtré et l'évaporation de l'éthanol fournit un solide qui recristallise dans l'éthanol en donnant 3,1g (70%) d'aiguilles incolores, F = 143° C.

Analyse % calculé pour $C_{13}H_{14}N_2O_2$: C 67,8 H 6,0 N 12,1
Trouvé: 67,6 5,9 11,9

Etape c) Méthyl-6 méthoxy-7 pyrido[4,3-b]benzofuranne (composé 9a, formule IX, R' = $CH_3$, Y'' = $CH_3$)

L'aminopyridine obtenue à l'exemple 2 8b (2,3g, 10 mmoles) dans l'acide chlorydrique 0,66N (30 ml) est agitée à 0°C et traitée par une solution aqueuse de nitrite de sodium (0,7g) additionnée goutte à goutte en dessous de 0°C. Après 15 minutes d'agitation à 0°C.la solution du dérivé diazoïque 8c ainsi formée est ajoutée goutte à goutte à un mélange d'acétone (50 ml), d'eau (12,5 ml) et de chlorure cuivrique (3,3g) chauffé à 35-40°C et maintenu sous violente agitation. Après avoir maintenu l'ensemble pendant 15 minutes à cette température, le mélange est chauffé à reflux pendant 5 minutes, refroidi et alcalinisé par addition d'une solution de soude. Après extraction au chloroforme et lavage à l'eau, le résidu de l'évaporation du solvant est recristallisé dans l'hexane pour donner 360 mg (16,9%) d'aiguilles incolores, F = 141-143°C.

Analyse % calculé pour $C_{13}H_{11}NO_2$: C 73,2 H 5,2 N 6,5
Trouvé: 72,9 5,2 6,3

Etape d) Méthyl-6 hydroxy-7 pyrido[4,3-b]benzofuranne (composé 9b, formule IX, R' = $CH_3$, Y'' = H)

Le composé 9a obtenu à l'exemple 3 (2,5g, 11,7 mmoles) dans le chlorure de pyridinium anhydre (25g) est chauffé à 220-230°C pendant 15 minutes et le mélange est versé dans l'eau glacée. Le solide résultant est filtré et recristallisé dans l'éthanol pour donner 1,8 g (77%) du chlorhydrate de 9b, F > 300°C. Ce chlorhydrate est libéré quantitativement en le dissolvant dans l'eau bouillante et en le traitant par de l'ammoniaque en excès. Le composé 9b recristallise dans le toluène en aiguilles incolores, F = 260-263° C.

Analyse % calculé pour $C_{12}H_9NO_2$: C 72,3 H 4,5 N 7,0
Trouvé: 72,5 4,7 7,0

Etape e) Méthyl-6 hydroxy-7 formyl-8 pyrido[4,3-b]- benzofuranne (composé 10a, formule X, R' = $CH_3$)

Le mélange formé par le dérivé hydroxylé 9b (5g, 25,1 mmoles), l'hexaméthylènetétramine (5,25g) et l'acide acétique (75ml) est chauffé à reflux pendant 3 h. Après addition de 100 ml d'acide chlorhydrique 3N, le nouveau mélange est chauffé à reflux pendant encore 3 h et le solvant est évaporé. Le résidu est repris dans l'eau, neutralisé par une solution saturée d'hydrogènocarbonate de sodium et le précipité obtenu, filtré et séché, recristallise dans le méthanol en donnant 2,1g (36%) d'aiguilles jaunes, F = 212°C, correspondant à l'hémihydrate du composé 10a.

Analyse % calculé pour $C_{13}H_9NO_3$, $1/2H_2O$: C 66,1 H 4,2 N 5,9
Trouvé: 65,9 4,0 5,8

Etape f) Méthyl-6 carbéthoxy-9 pyrido[3',4':4,5]furo- [3,2-g]coumarine (composé 11a, formule XI, R' = $CH_3$, Y''' = COO $C_2H_5$

L'aldéhyde 10a (2g) est chauffé au reflux dans l'éthanol (10 ml) avec le diéthylmalonate (2,28 g) et la pipéridine (0,2 ml) pendant 20 minutes. Le mélange réactionnel refroidi fournit un solide qui recristallise dans l'éthanol en donnant 2,6 g (91%) d'aiguilles jaunes F = 256-258° C.

Analyse % calculé pour $C_{18}H_{13}NO_5$: C 66,8 H 4,0 N 4,3
Trouvé: 66,6 4,0 3,9

Etape g) Méthyl-6 pyrido[3',4':4,5]furo[3,2-g] coumarine (composé 5a, formule I, R = H, R' = $CH_3$)

La carbéthoxy-9 méthyl-6 pyrido[3',4':4,5] furo[3,2-g] coumarine 11a (2,6g, 8 mmoles) est chauffée à reflux pendant 2 h dans le mélange acide acétique (15,6 ml)-acide chlorhydrique (14 ml) et le mélange est refroidi. Le solide formé est filtré et séché pour donner 2,2 g de l'acide 11b sous la forme de son chlorhydrate. Ce dernier est dissous dans l'eau chaude, alcalinisé par l'ammoniaque et neutralisé par l'acide acétique. Le solide résultant est filtré et séché pour fournir 1,9 g (80%) d'un solide F > 300°C. Cet acide (500 mg) mélangé avec l'oxyde cuivrique (5 mg) et l'o-phénanthroline (5 mg) est chauffé à 200-220°C dans la quinoléine (2,7 ml) pendant 20 minutes, puis pendant 5 minutes à 240°C. Le mélange refroidi fournit un solide qui recristallise dans le toluène en donnant 210 mg (49%) du composé pur, énoncé en tête, F = 295-302°C.

Analyse % calculé pour $C_{15}H_9NO_3$: C 71,7 H 3,6 N 5,5
Trouvé: 71,7 3,7 5,3

**EXEMPLE 2**

Etape a) N-méthyl O-(diméthyl-4',8' coumarinyl)-7 pipe- ridone-4 oxime (composé de formule IV R = R' = Y = $CH_3$)

A un mélange de 850 mg (3,92 mmoles) d'hydroxylamine de formule

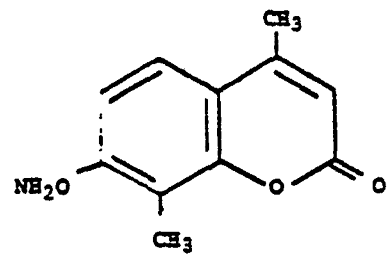

et de chlorhydrate de méthyl-1-pipéridone-4 dans 40 ml d'éthanol, on ajoute lentement, en agitant, 15 gouttes d'acide chlorhydrique concentré. Après quelques minutes, le mélange commence à se dissoudre puis précipite. Après 3 h 30 d'agitation le précipité est essoré, lavé à l'alcool et séché. On obtient 1,24 g (3,6 mmoles) (94%) de chlorhydrate du composé du titre, F = 204-205° C.300 mg de ce chlorhydrate en suspension dans 6 ml d'eau sont neutralisés par une solution saturée d'hydrogénocarbonate de sodium. Après 0,5 h d'agitation, le précipité formé est essoré, lavé à l'eau et séché. Après deux cristallisations dans l'éthanol, on obtient 120 mg de base, F = 138° C dec.

Analyse % calculé pour $C_{17}H_{20}N_2O_3$: C 67,98 H 6,71 N 9,33

Trouvé: 67,65 6,73 9,27

Etape b) Tétrahydro-1,2,3,4 triméthyl-2,6,10 pyrido-[3',4':4,5]furo[3,2-g]coumarine (composé 14b de formule V avec R = R' = $CH_3$ et Y = $CH_3$)

2,28 g (6,78 mmoles) de l'oxime préparée à l'exemple 2, etape a), sont mis en suspension dans 60 ml d'acide acétique contenant 7% d'acide chlorhydrique sec. Le mélange est chauffé à 80° C sous un léger courant d'acide chlorhydrique pendant 5 h. Après refroidissement, le précipité formé est essoré, lavé à l'acide acetique et séché. On obtient 1,600 g (4,54 mmoles) (67%) de chlorhydrate du composé du titre F = 268-271° C déc.

1,50 g de ce chlorhydrate en suspension dans 5 ml d'eau, sont neutralisés par une solution saturée d'hydrogénocarbonate de sodium. Après 0,5 h d'agitation, le précipité essoré, lavé à l'eau, séché et cristallisé deux fois dans l'alcool, fournit 66 mg de base, F = 201-202° C. RMN($CDCl_3$, δppm, 60 MHz), 2,5 (3H, s, $CH_3$); 2,58 (6H, s, $CH_3$); 2,9 (4H,s large, $CH_2$ en 3 et 4); 3,63 (2H, s large, $CH_2$ en 1); 6,26 (1H, m, $H_9$); 7,43 (1H, S, $H_{11}$)

Analyse % calculé pour $C_{17}H_{17}NO_3$: C 72,06 H 6,05 N 4,94

Trouvé: 71,85 6,05 4,91

Etape c) Dans les mêmes conditions qu'à l'exemple 11, l'aromatisation de 248 mg (0,77 mmoles) du composé obtenu à l'exemple 9 fournit 109 mg de produit brut. Après deux recristallisations du mélange $CH_2Cl_2$/EtOH on obtient 77 mg (37%) du composé 5b de formule I avec R = $CH_3$ et R' = $CH_3$.

EXEMPLE 3

Etapes a) et b) Tétrahydro 1,2,3,4 diméthyl-6,10 pyrido- [3',4':4,5]furo[3,2-g]coumarine (composé 14a de formule V avec R = R' = $CH_3$, Y = H

Le composé du titre, été préparé selon le procédé décrit pour le composé de l'exemple 9 à partir de l'hydroxylamine et du chlorhydrate de piperi-done-4.

L'oxime intermédiaire n'a pas été caractérisée. A partir de 1,360 g (6,63 mmoles) d'hydroxylamine, on obtient 1,150 g de chlorhydrate brut du composé du titre (56,8%), F = 285-290° C.

La base libre correspondante recristallisée dans l'alcool, se présente sous la forme d'aiguilles incolores, F = 211-212° C.

SM m/c 269 (M+), 240, 212

RMN ($CDCl_3$, δppm, 60 MHz); 1,73 (1H, s, $H_2$); 2,43 (3H,d, $CH_3$ en 10); 2,53 (3H,s,$CH_3$ en 6); 2,83 (2H,m, $CH_2$ en 3); 3,2 (2H,m,$CH_2$ en 4); 3,96 (2H,m,$CH_2$ en 1); 6,16 (1H,m,$H_9$); 7,33 (1H,s,$H_{11}$).

Analyse % calculé pour $C_{16}H_{15}NO_3$, 1/2 $H_2O$: C 69,05 H 5,80 N 5,03

Trouvé: 68,87 5,92 4,77

Etape c) Diméthyl-6,10 pyrido[3',4':4,5]furo[3,2-g]- coumarine (composé 5b de formule I avec R = $CH_3$ et R' = $CH_3$)

404 mg (1,32 mmoles) du chlorhydrate obtenu à l'exemple 10 sont mis en suspension dans 15 ml de décaline et chauffés au reflux, en présence de 300 mg de palladium sur charbon à 10% pendant 5 h. Le Pd/C est filtré à chaud, lavé avec 5 ml de décaline chaude. Après refroidissement, les cristaux formés sont essorés et séchés.

On obtient 130 mg du composé du titre qui cristallisent d'un mélange $CH_2Cl_2$/EtOH sous forme d'aiguilles incolores, F=270-271°C.

SM: m/c 265 (M+), 237, 118

RMN($CDCl_3$, δppm, 100 MHz), 2,58 (3H, d, J=1,2Hz, $CH_3$ en 10), 2,67 (3H, d, J=0,5 Hz, $CH_3$ en 6); 6,34 (1H, d, J=1,2 Hz, $H_9$); 7,59 (1H, dxd, J=0,9 Hz, J=5,7 Hz, $H_4$); 8,08(1H, s, $H_{11}$); 8,70(1H, d, J=5,7 Hz, $H_3$); 9,29(1H, d, J=0,9 Hz, $H_1$).

Analyse % calculé pour $C_{16}H_{11}NO_3$: C 72,44 H 4,18 N 5,28

Trouvé: 72,13 4,25 4,71

«Etape c) Dans les mêmes conditions qu' à l'exemple 11, l'aromatisation de 248 mg (0,77 mmoles) du composé obtenu à l'exemple 9 fournit 109 mg de produit brut. Après deux recristallisations du mélange $CH_2Cl_2$/EtOH on obtient 77 mg (37%) du composé 5b de formule I avec R=$CH_3$ et R'=$CH_3$.»

Les nouveaux composés de l'invention ont été soumis à des essais permettant d'apprécier leur activité en cosmétologie et à titre de médicament.

Dans ce qui suit, les composés de formule I selon l'invention dans laquelle R'=$CH_3$ et R=H ou R'=$CH_3$ et R=$CH_3$ seront respectivement désignés par les références abrégées 5a et 5b.

Les composés 5a et 5b ainsi que certains dérivés intermédiaires produits au cours de leur synthèse (composés 14a et 14b) ont été étudiés du point de vue de leurs propriétés photophysiques, photochimiques et de leur réactivité in vitro vis-à-vis de l'ADN. Ces études ont d'abord essentiellement concerné leurs propriétés spectroscopiques en solution: 1) Absorption et notamment absorption du rayonnement à 365 nm, c'est-à-dire à la longueur d'onde d'irradiation généralement utilisée pour la plupart des études photobiologiques; 2) Emission; 3) Stabilité photochimique lors d'une irradiation ultraviolette (UV-A) à des doses comparables à celles utilisées lors des expériences photobiologiques. Dans une deuxième étape, l'affinité de ces molécules vis-à-vis de l'ADN, puis leur photoréaction lors de l'irradiation des complexes ADN -pyrido psoralènes ont été testées (expériences de fusionrenaturation de l'ADN).

Le composé 5b possède un coéfficient d'absorption moléculaire à 365 nm nettement inférieur à celui du psoralène.

La molécule libre du composé 5b en solution présente une bonne stabilité photochimique vis-à-vis du rayonnement UV-A. Les expériences de dénaturationrenaturation thermique de l'ADN modifié par ce composé en présence d'UV-A conduisent à des valeurs de fraction non renaturante voisines de 100% jusqu'à la dose incidente de 27 kJ/m$^2$.

Il résulte de ces études in vitro que les composés 5a et 5b sont capables de ne former que des monoadditions sur l'ADN.

Le caractère monofonctionnel des nouveaux composés 5a et 5b a été encore confirmé par les expériences suivantes:

1) Utilisation de mutants de levure bloqués spécifiquement dans la réparation des pontages interbrins de l'ADN:

On rappelle que la photoaddition de furocoumarines bifonctionnelles induit à la fois des pontages interbrins et des monoadditions sur les bases de l'ADN. Celle de furocoumarines monofonctionnelles produit uniquement cette dernière réaction.

On dispose d'un mutant de levure, pso2, qui a les propriétés suivantes: a) Il est beaucoup plus sensible que le type sauvage dont il dérive à l'effet létal de la photoaddition des psoralènes bifonctionnels (Isolation and characterization of pso mutants sensitive to photoaddition of psoralen derivatives in Saccharomyces cerevisiae: J.A.P. HENRIQUES and E. MOUSTACCHI. Genetics 95, 273-288 (1980)), ainsi qu'à d'autres agents de pontage, tels que les moutardes azotées bifonctionnelles (Mutagenesis induced by mono and bifunctional alkylating agents in yeast mutants sensitive to photoaddition of furocoumarins (pso). CASSIER et E. MOUSTACCHI, Mutation Res. 84, 37-47 (1981)), ou la mitomycine C. b) Il est relativement très peu sensible par rapport au produit sauvage à la photoaddition de psoralènes monofonctionnels du type 3-carbéthoxypsoralène ou aux moutardes monofonctionnelles. De même pso2 présente la même sensibilité que le type sauvage aux ultraviolets de 254 nm ou aux radiations ionisantes qui sont connus pour produire essentiellement des lésions des brins et des ruptures de l'ADN sans production de pontages aux doses biologiquement significatives. c) On a démontré biochimiquement que le mutant pso2 est bloqué dans la réparation des pontages interbrins de l'ADN (The fate of 8-methoxypsoralens photo-induced cross-links in nuclear and mitochondrial yeast DNA. Comparison of wild type and repair-deficient strains: N. MAGANA-SCHWENCKE, J.A.P. HENRIQUES, R. CHANET and E. MOUSTACCHI. Proc. Natl. Acad. Sci. (USA), (1981)). Ce blocage est spécifique car le mutant pso2 répare comme le type sauvage les monoadditions photoinduites sur l'ADN.

Autrement dit si le mutant pso2 se montre plus sensible que le type sauvage à l'effet létal d'un agent, on peut en déduire que cet agent produira des pontages interbrins de l'ADN, et qu'il s'agit d'un composé bifonctionnel. En revanche, si le mutant pso2 a la même sensibilité que le type sauvage à un agent donné on en conclut cue cet agent ne provoque pas de pontage et qu'il est par conséquent de type monofonctionnel.

Les expériences montrent que le mutant pso2 est 3 à 4 fois plus sensible que la souche sauvage isogénique aux produits 14a et 14b. Ces derniers sont donc de type bifonctionnel.

Par contre on constate que le mutant pso2 a la même sensibilité que le type sauvage à la photoaddition du produit 5b. Le mutant ayant la même capacité que le type sauvage à réparer les lésions de monoaddition, il est

clair que le composé 5b est de type monofonctionnel. On confirme par ailleurs sur les souches de levure que les nouveaux composés 5a et 5b sont très photoréactifs.

2) Vérification biochimique directe de l'absence de pontage dans l'ADN de cellules traitées par le produit 5b plus une irradiation à 365 nm.

La validité des conclusions précédentes est confirmée par l'analyse biochimique in vivo. En effet l'ADN de cellules de type sauvage a été extrait immédiatement après le traitement avec le produit 5b à concentration $10^{-5}$M plus une irradiation par deux doses de radiations de 365 nm (30% et 5% de survie). Après séparation de l'ADN nucléaire de l'ADN mitochondrial en gradient de densité de chlorure de Cesium, l'ADN est cassé de manière à avoir des segments de taille homogène (1 pontage en moyenne par molécule pour le 8-méthoxypsoralène), il est dénaturé puis renaturé. Si l'ADN contient des ponts interbrins il se renature et se retrouve sous la forme double chaîne séparable en gradient de densité de l'ADN simple chaîne. Par contre quand l'ADN ne contient pas de pontages, il reste sous forme simple chaîne après renaturation. C'est cette dernière situation que l'on observe expérimentalement avec le produit 5b. Les expériences de contrôle avec le 8-méthoxypsoralène effectuées dans les mêmes conditions mettent en évidence de l'ADN ponté en double brin. Pour les détails de la technique utilisée voir réf: "The fate of 8-methoxypsoralens pphoto-induced cross-links in nuclear and mitochondrial yeast DNA. Comparison of wild type and repair-deficient strains" N. MAGANA-SCHWENCKE, J.A.P. HENRIQUES, R. CHANET and E. MOUSTACCHI. Proc. Natl. Acad. Sci. (USA) (1981), et "Absence de pontages interchaînes dans l'ADN traité par le 3-carbéthoxypsoralène et une irradiation à 365 nm". N. MAGANA-SCHWENCKE, D. AVERBECK, J.A.P. HENRIQOES et E. MOUSTACCHI. C.R. Acad. Sci. Paris 291, 207-210 (1980).

En résumé l'utilisation du mutant pso2 et l'examen direct de l'ADN de cellules traitées in vivo montrent clairement que le produit 5 b est bien de type monofonctionnel.

Les méthodes utilisées pour apprécier l'activité photobiologique des nouveaux dérivés du psoralène selon l'invention sont notamment décrites dans les références bibliographiques ci-après:

. AVERBECK D., BISAGNI E., MOUSTACCHI E. (1978), Biochim. Biophys. Acta 518, 464.
. AVERBECK D., MOUSTACCHI E. (1979) Mutation Res. 68, 133.
. AVERBECK D., MOUSTACCHI E. (1980) Photochem. Photobiol. 31, 475.
. AVERBECK D., AVERBECK S., DALL'ACQUA, F. (1981) Il Farmaco 36, 492.

Pour la détection de l'activité photobiologique, le système eukaryote unicellulaire de la levure Saccharomyces cerevisiae s'est révélé très utile, voir AVERBECK, D. (1981) dans Trends in Photobiology, Proc. of the 8th International Congress in Photobiology 20-25 july 1980 Strasbourg, Eds. C. Helène, M. Charlier, Th. Montenay-Garestier, G. Laustriat, Plenum Publishing Corporation, New York. Cette activité photobiologique a en effet été définie par l'induction d'effets létaux, l'induction de mutations cytoplasmiques "petite colonie" (dommages dans l'ADN mitochondrial) et l'induction de mutations nucléaires (réverses et aller). Les expériences ont été faites selon les méthodes habituellement utilisées et décrites dans les articles sus-indiqués.

Dans les essais sur l'induction des effets létaux chez la levure exprimés par l'inhibition de la capacité des cellules de former une colonie, on a observé qu'en présence de radiation à 365 nm (UVA) les composés 14a et 14b a des concentrations équimolaires ($5.10^{-5}$M) manifestent une activité comparable à celle du 8-MOP, agent bifonctionnel largement utilisé en PUVA-thérapie. En fonction de la dose d'UVA et des survivants, ces composés montrent aussi, en ce qui concerne l'induction de mutations cytoplasmiques, une activité comparable à celle du 8-MOP, donc une photoréactivité semblable avec l'ADN et une activité de type bifonctionnel.

Les composés 5a et 5b se sont avérés des agents de type monofonctionnel, et leur activité photoinduite antiproliférative voisine, dans le cas du composé 5a de celle du bifonctionnel 8-MOP et dans le cas du composé 5b de celle du carbéthoxy-3 psoralène (3-CPs), offre un grand intérêt.

Contrairement au 8-MOP les deux composés ont par dose d'UVA un pouvoir d'induction de mutations cytoplasmiques "petite colonie" élevé indiquant une photoaffinité importante vers l'ADN. En fonction des survivants cette efficacité d'induction est comparable à celle du 3-CPs ce qui est en accord avec la notion de monofonctionnalité.

Il est important de noter que pour l'induction de mutations núcléaires réverses (his+), et aller ($can^R$) en fonction de la dose d'UVA le composé 5a est bien moins mutagène que les furocoumarines bifonctionnelles (8-methoxypsoralène, 5-métho-xypsoralène et 4,5',8-triméthylpsoralène) habituellement utilisées en photochimiothérapie. En fonction du nombre de survivants, ce qui permet d'apprécier l'efficacité relative des composés sur l'induction de mutations, ce composé s'est montré nettement moins mutagène que le 8-MOP mais d'une efficacité comparable à celle du 3-CPs.

Le fait que le composé 11a de formule XI se soit avéré complètement inactif dans les différents essais photobiologiques dans les conditions expérimentales et les gammes de doses utilisées habituellement, montre que le groupement carbéthoxy en position 3 annule l'induction de photoadditions à l'ADN et l'effet photosensibilisateur des composés décrits.

L'activité clinique et thérapeutique du composé 5b a été mesurée sur la peau humaine.

On a dilué 10 mg du composé 5b dans 2 g d'hydrocérine ROC (Lanoline purifiée) rendu liquide par un chauffage à 60°C.

Cette preparation topique a été testée sur une surface ronde de 3 cm de diamètre au centre d'une plaque de psoriasis lombaire. La quantité appliquée a été d'environ 30 µg/$cm^2$ de produit 5b. L'application a été faite deux

heures avant l'irradiation (UVA) afin d'assurer une pénétration cutanée suffisante. Après ces deux heures la dose d'irradiation a été d'emblée de 5 J/cm² et les séances se sont repetees les lundi, mercredi et vendredi jusqu'à l'apparition d'un effet thérapeutique. Des la 7eme séance (dose d'irradiation totale 35 J/cm²) un blanchiment était observé. Parallèlement, une pigmentation était apparue.

Aucun érytheme ne s'est manifesté avec le produit 5b. La zone témoin, traitée avec la lanoline seule, n'a montré ni amélioration ni pigmentation.

En conclusion, les produits monofonctionnels 5a et 5b ont des caractéristiques photobiologiques très favorables à un emploi en photochimiothérapie du fait de leur bonne activité antiproliférative non accompagnée de risques mutagènes importants. Les essais réalisés sur la peau humaine montrent que ces composés possèdent une activité antipsoriasique et pigmentogène.

**Revendications** pour les Etats contractants

BE CH DE GB IT LI LU NL SE

1. Pyrido[3,4-h]psoralènes ou pyrido-[3',4':4,5]furo[3,2-g] coumarines répondant à la formule I

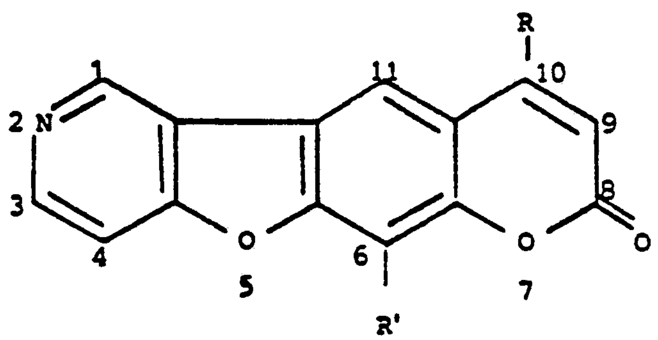

(I)

formule dans laquelle R est un atome d'hydrogène ou un groupe alkyle en $C_1$ -$C_4$ et R' est un groupe méthyle ou méthoxy.

2. Composés selon la revendication 1, dans lesquels R est un groupe méthyle ou un atome d'hydrogène.

3. Composés selon l'une des revendications 1 ou 2 dans lesquels R' est un groupe méthyle.

4. Composé selon la revendication 1, dans lequel R est un atome d'hydrogène et R' est un groupe méthyle.

5. Composé selon la revendication 1, dans lequel R et R' représentent tous deux un groupe méthyle.

6. Procédé pour l'obtention des composés selon l'une quelconque des revendications 1 à 5, caractérisé par les étapes suivantes:

(a) on fait réagir une amino oxy-7 coumarine de formule II

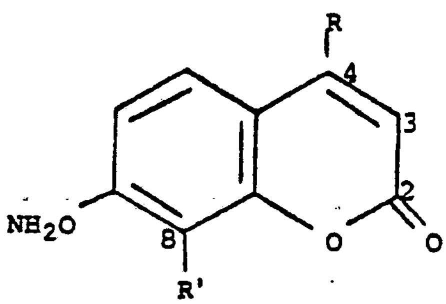

(II)

dans laquelle R et R' ont la signification susindiquée, sur une 4-pipéridone de formule III

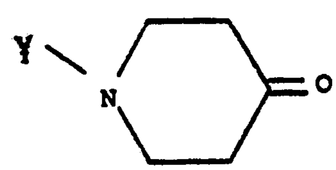

(III)

dans laquelle le substituant Y peut être un atome d'hydrogène, ce qui conduit à une oxime de formule IV

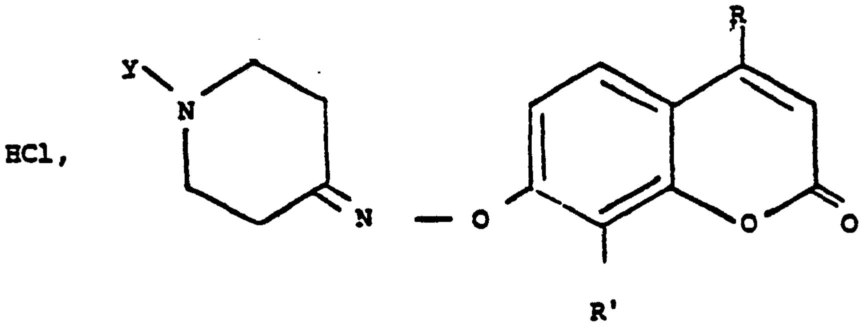

(IV)

dans laquelle R, R' et Y ont les significations indiquées ci-dessus;

(b) on réalise la cyclisation de l'oxime IV en milieu acide, ce qui conduit à un tétrahydro-1,2,3,4 pyrido[3',4':4,5]psoralène de formule V

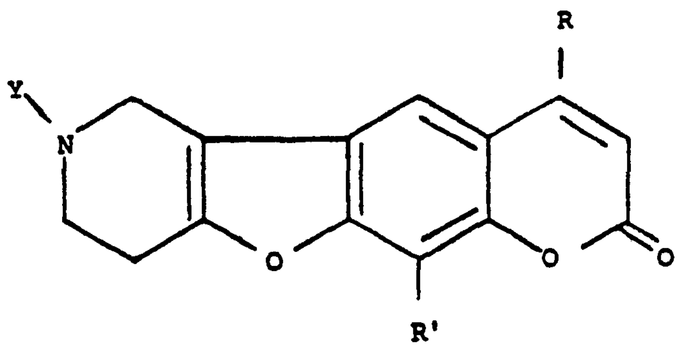

(V)

dans laquelle R, R' et Y ont la signification susindiquée;

(c) on effectue l'aromatisation du produit V en présence d'un agent de déshydrogénation, pour obtenir finalement le composé désiré de formule (I).

7. Procédé pour l'obtention des composés selon l'une quelconque des revendications 1 à 4, dans lesquels R est un atome d'hydrogène et R' un radical méthyle ou méthoxy, ledit procédé étant caractérisé par les étapes suivantes:

(a) on fait réagir de l'éther monométhylique de la méthyl-2 ou méthoxy-2 résorcine de formule VI

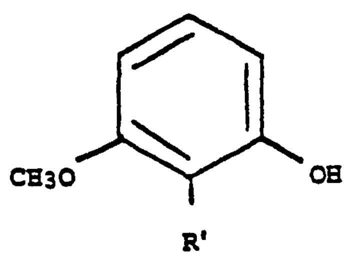

(VI)

dans laquelle R' = $CH_3$ ou $OCH_3$ sur la nitro-3 chloro-4 pyridine de formule VII

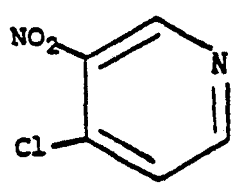

(VII)

ce qui conduit à un dérivé nitré intermédiaire répondant à la formule VIII

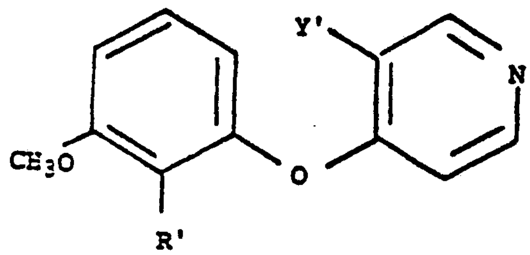

(VIII)

dans laquelle R' est $CH_3$ ou $OCH_3$ et Y' = $NO_2$;

(b) on transforme le produit nitré (VIII dans lequel Y' = $NO_2$), en l'amine correspondante dans laquelle Y' = $NH_2$, laquelle est ensuite diazotée pour fournir le produit correspondant avec Y' = $N_2^+Cl^-$;

(c) on cyclise ce dernier produit pour obtenir le composé de formule IX

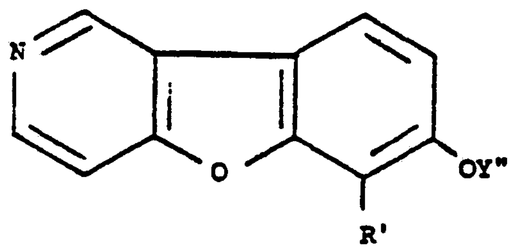

(IX)

dans laquelle Y'' représente $CH_3$ et R' a la signification indiquée ci-dessus, à savoir $CH_3$ ou $OCH_3$;

(d) on soumet le produit intermédiaire IX à une réaction de déméthylation, pour fournir le composé correspondant de formule IX dans laquelle Y'' est un atome d'hydrogène, à savoir un hydroxypyridobenzofuranne;

(e) on soumet ce dernier composé à une réaction de formylation pour obtenir le produit intermédiaire de formule X

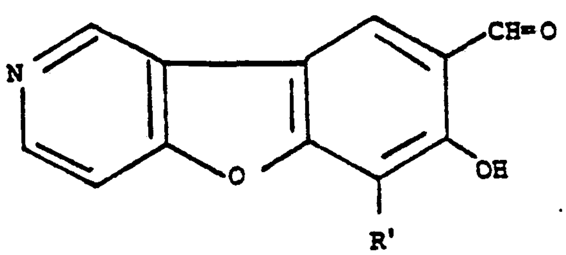

(X)

dans laquelle R' représente un radical méthyle ou méthoxy;

(f) on condense l'aldéhyde X avec le diéthylmalonate ce qui conduit à un carbéthoxypyrido-psoralène de formule XI

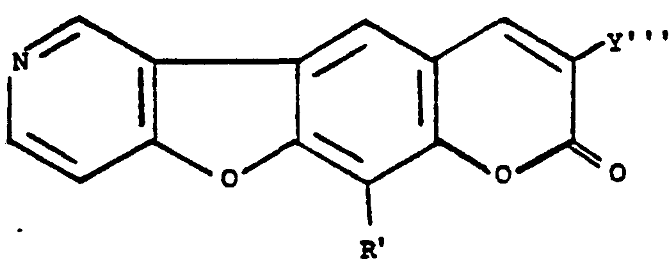

(XI)

dans laquelle Y''' représente le radical $COOC_2H_5$ et R' = $CH_3$ ou bien $OCH_3$;

(g) on hydrolyse le produit XI pour obtenir l'acide correspondant (Y''' = COOH);

(h) on effectue la décarboxylation de cet acide, ce qui permet d'obtenir le pyridopsoralène de formule I dans laquelle R = H et R' = $CH_3$ ou bien $OCH_3$.

8. A titre de produits intermédiaires pour la mise en oeuvre du procédé selon la revendication 6, les dérivés de psoralène de formule

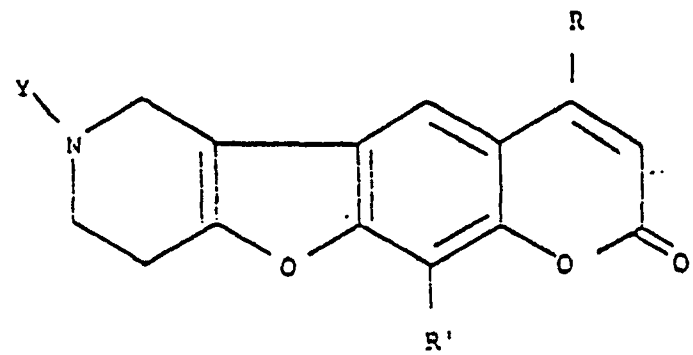

dans laquelle R et R' ont la signification indiquée à l'une quelconque des revendications 1 à 5 et Y est un atome d'hydrogène, un radical méthyle ou un groupe benzyle ainsi que les sels de ces dérivés, en particulier les chlorhydrates.

9. Produit selon la revendication 8, dans lequel R et R' représentent tous deux un radical méthyle et Y est un atome d'hydrogène.

10. Produit selon la revendication 8, dans lequel R et R' représentent tous deux un radical méthyle et Y est un groupe méthyle.

11. Composition pharmaceutique contenant à titre d'agent actif, au moins un des produits selon l'une des revendications 1, 2, 3, 4, 5, 8, 9 et 10.

12. Composition pharmaceutique selon la revendication 11 pour le traitement des affections cutanées, et plus spécialement pour le traitement des dermatoses inflammatoires, bénignes et malignes, à savoir le psoriasis, le mycosis fongoïde, les eczémas constitutionnels et de contact, les parapsoriasis en plaques et en gouttes, les pelages, les prurigas, les lichens plans, les urticaires pigmentaires ainsi que les troubles de la pigmentation et les photodermatoses.

13. Composition pharmaceutique selon l'une des revendications 11 ou 12 dans laquelle l'agent actif est associé à un véhicule pharmaceutiquement acceptable, pour une administration locale ou orale.

14. Composition selon la revendication 13, caractérisée en ce que l'agent actif se trouve dans la composition, à raison de 0,1 à 2% en poids par rapport au poids total de la composition.

15. Composition cosmétique contenant, à titre d'agent actif, au moins un des produits selon l'une des revendications 1, 2, 3, 4, 5, 8, 9 et 10 ou obtenus par les procédés des revendications 6 ou 7, en association avec un véhicule convenant à l'application externe.

16. Composition selon la revendication 15, caractérisée en ce qu'elle contient une quantité efficace pour stimuler la pigmentation de la peau, d'au moins un des composés selon l'une des revendications 1 à 5.

**Revendications** pour l'Etat contractant AT

1. Procédé pour l'obtention des composés de formule

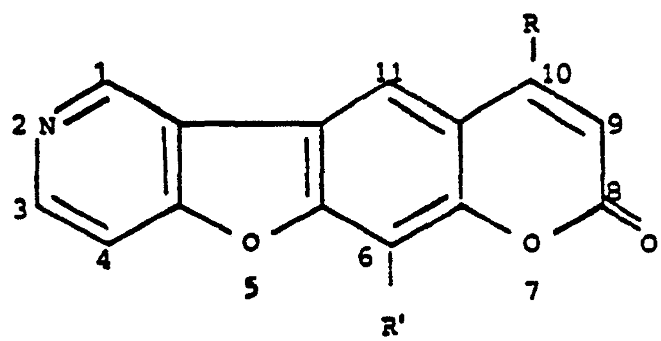

(I)

dans laquelle R est un atome d'hydrogène ou un groupe alkyle en $C_1$-$C_4$ et R' est un groupe méthyle ou méthoxy, caractérisé par les étapes suivantes:

a) on fait réagir une aminooxy-7 coumarine de formule II

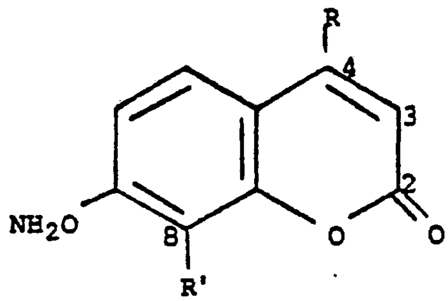

(II)

dans laquelle R et R' ont la signification sus-indiquée, sur une pipéridone-4 de formule III

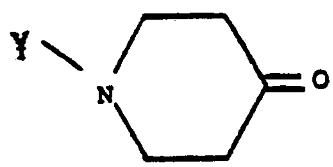

(III)

dans laquelle le substituant Y peut être un atome d'hydrogène, ce qui conduit à une oxime de formule IV

HCl, 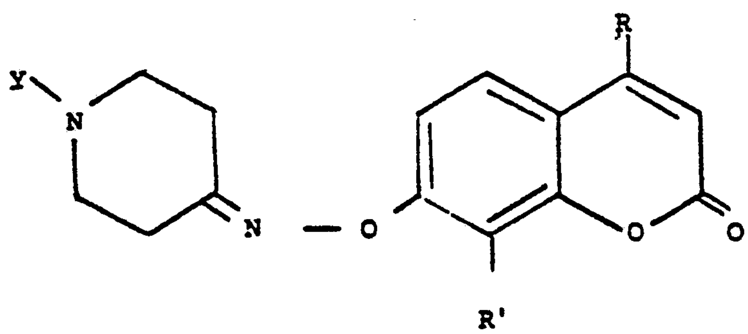

(IV)

dans laquelle R, R' et Y ont les significations indiquées ci-dessus;

(b) on réalise la cyclisation de l'oxime IV en milieu acide, ce qui conduit à un tétrahydro-1,2,3,4 pyrido[3',4':4,5]psoralène de formule V

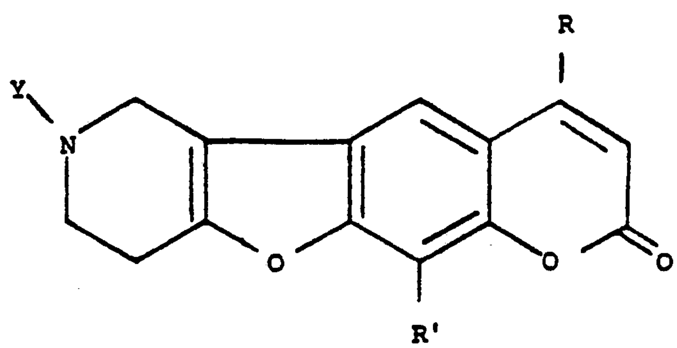

(V)

dans laquelle R, R' et Y ont la signification sus-indiquée;

c) on effectue l'aromatisation du produit V en présence d'un agent de déshydrogénation, pour obtenir finalement le composé désiré de formule I.

2.Procédé pour l'obtention des composés répondant à la formule I indiquée à la revendication 1, dans laquelle R est un atome d'hydrogène et R' un radical méthyle ou méthoxy, caractérisé par les étapes suivantes:

(a) on fait réagir de l'éther monométhylique de la méthyl-2 ou méthoxy-2 résorcine de formule VI

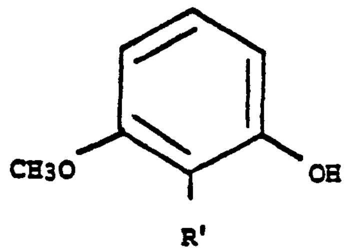

(VI)

dans laquelle R' = $CH_3$ ou $OCH_3$ sur la nitro-3 chloro-4 pyridine de formule VII.

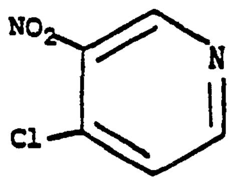

(VII)

ce qui conduit à un dérivé nitré intermédiaire répondant à la formule VIII

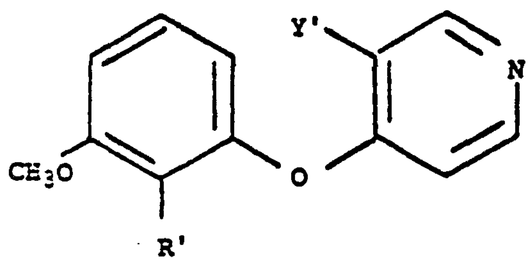

(VIII)

dans laquelle R' est $CH_3$ ou $OCH_3$ et Y' = $NO_2$;

(b) on transforme le produit nitré (VIII dans lequel Y' = $NO_2$), en l'amine correspondante dans laquelle Y' = $NH_2$, laquelle est ensuite diazotée pour fournir le produit correspondant avec Y' = $N_2^+ Cl^-$;

(c) on cyclise ce dernier produit pour obtenir le composé de formule IX

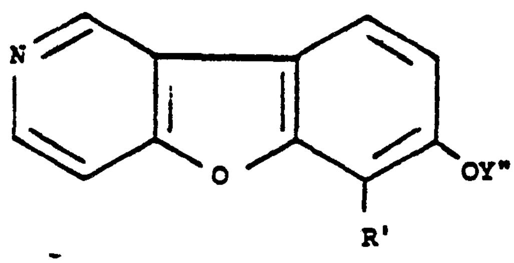

(IX)

dans laquelle Y'' représente $CH_3$ et R' a la signification indiquée ci-dessus, à savoir $CH_3$ ou $OCH_3$;

(d) on soumet le produit intermédiaire IX à une réaction de déméthylation, pour fournir le composé correspondant de formule IX dans laquelle Y'' est un atome d'hydrogène;

(e) on soumet ce dernier composé à une réaction de formylation pour obtenir le produit intermédiaire de formule X

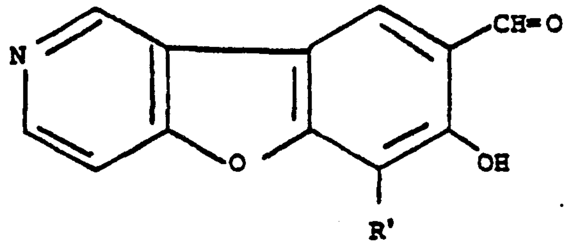

(X)

dans laquelle R' représente un radical méthyle ou méthoxy;
(f) on condense l'aldéhyde X avec le diéthylmalonate ce cui conduit à un composé de formule XI;

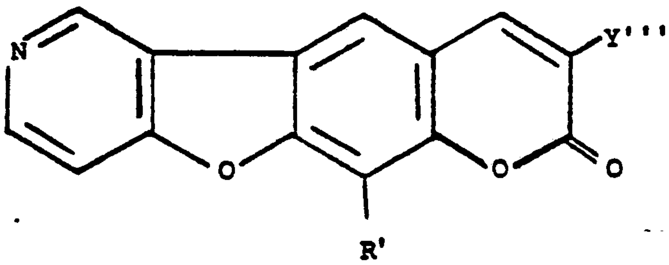

(XI)

dans laquelle Y''' représente le radical $COOC_2H_5$ et R' = $CH_3$ ou bien $OCH_3$;
(g) on hydrolyse le produit XI pour obtenir l'acide correspondant (Y''' = COOH);
(h) on effectue la décarboxylation de cet acide, ce qui permet d'obtenir le composé de formule I dans laquelle R = H et R' = $CH_3$ ou bien $OCH_3$.

3. Procédé selon l'une des revendications 1 ou 2 pour obtenir un composé dans lequel R est H et R' est $CH_3$.

4. Procédé selon la revendication 1 pour obtenir un composé dans lequel R et R' représentent $CH_3$.

5. Procédé de préparation d'une composition pharmaceutique caractérisé en ce qu'on mélange avec un excipient pharmaceutiquement acceptable une quantité thérapeutiquement efficace d'un composé répondant à la formule I cidessus dans laquelle R et R' ont les significations données dans la revendication 1.

6. Procédé selon la revendication 5 pour lequel R est H ou $CH_3$ et R' est $CH_3$.

7. Procédé selon l'une des revendications 5 ou 6, caractérisé en ce que l'agent actif est présent à raison de 0,1 à 2% en poids de la composition.

8. Procédé de préparation d'une composition cosmétique pour application locale, caractérisé en ce qu'on mélange avec un véhicule convenable un composé répondant à la formule I ci-dessus dans laquelle R et R' ont les significations données à la revendication 1.

9. Procédé selon la revendication 8 pour lequel R est H ou $CH_3$ et R' est $CH_3$.

**Patentansprüche**

für die Vertragsstaaten: BE, CH, DE, GB, IT, LI, LU, NL, SE

1. Pyrido[3,4-h]psoralene oder Pyrido[3',4':4,5]furo[3,2-g]-cumarine entsprechend der Formel I

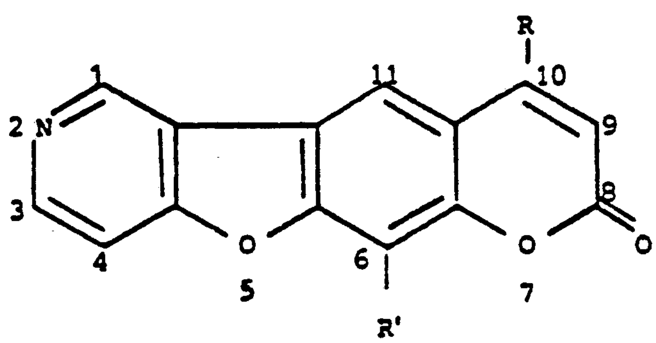

(I)

in der R ein Wasserstoffatom oder ein $C_1$-$C_4$-Alkylrest und R' ein Methyl- oder Methoxyrest ist.

2. Verbindungen nach Anspruch 1, worin R ein Methylrest oder ein Wasserstoffatom ist.

3. Verbindungen nach einem der Ansprüche 1 oder 2, worin R' ein Methylrest ist.

4. Verbindung nach Anspruch 1, worin R ein Wasserstoffatom und R' ein Methylrest ist.

5. Verbindung nach Anspruch 1, worin R und R' beide ein Methylrest sind.

6. Verfahren zur Herstellung der Verbindungen nach irgendeinem der Ansprüche 1 bis 5, gekennzeichnet

durch die folgenden Stufen

a) man setzt ein 7-Amino-oxycumarin der Formel II

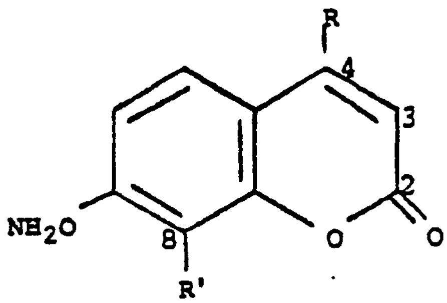

(II)

in der R und R' die vorstehend genannte Bedeutung haben, mit einem 4-Piperidon der Formel III

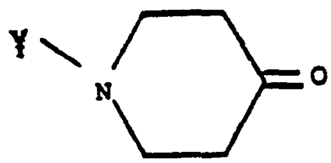

(III)

in der der Substituent Y ein Wasserstoffatom sein kann, um, was zu einem Oxim der Formel IV

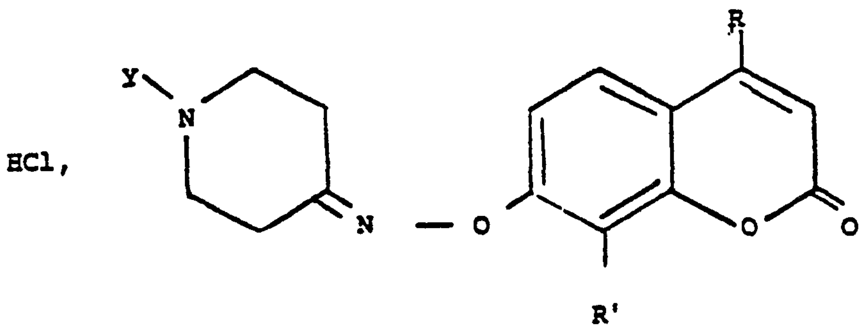

(IV)

in der R, R' und Y die vorstehend genannten Bedeutungen haben, führt;

b) man führt die Cyclisierung des Oxims IV in saurem Medium durch, was zu einem 1,2,3,4-Tetrahydropyrido[3',4':4,5]-psoralen der Formel V

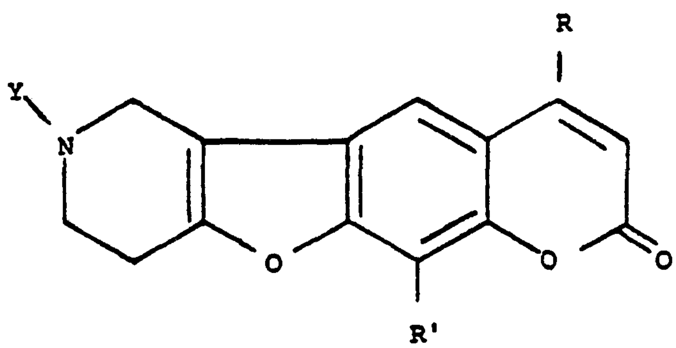

(V)

in der R, R' und Y die vorstehend genannte Bedeutung haben, führt

c) man führt die Aromatisierung des Produkts V in Gegenwart eines Dehydriermittels durch, um schließlich die gewünschte Verbindung der Formel I zu erhalten.

7. Verfahren zur Herstellung der Verbindungen nach irgendeinem der Ansprüche 1 bis 4, worin R ein Wasserstoffatom und R' ein Methyl- oder Methoxyrest ist, gekennzeichnet durch die folgenden Stufen:

a) man setzt Monomethylether von 2-Methyl- oder 2-Methoxyresorcin der Formel VI

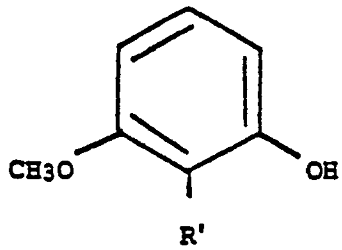

(VI)

in der R' = $CH_3$ oder $OCH_3$, mit 3-Nitro-4-chlorpyridin der Formel VII

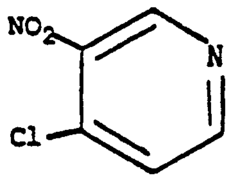

(VII)

um, was zu einem nitrierten Zwischenderivat entsprechend der Formel VIII

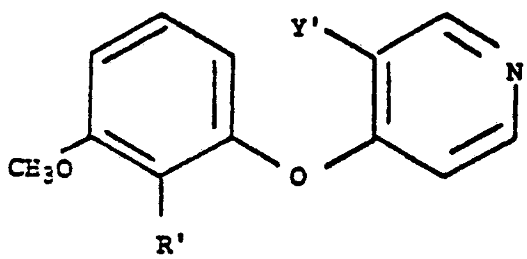

(VIII)

in der R' für $CH_3$ oder $OCH_3$ und Y' für $NO_2$ steht, führt;

b) man wandelt das nitrierte Produkt (VIII, worin Y' = $NO_2$), in das entsprechende Amin um, in dem Y' = $NH_2$, und diazotiert anschließend dieses Amin, um das entsprechende Produkt mit Y' = $N_2^+Cl^-$ zu liefern;

c) man cyclisiert das letztgenannte Produkt, um die Verbindung der Formel IX zu erhalten

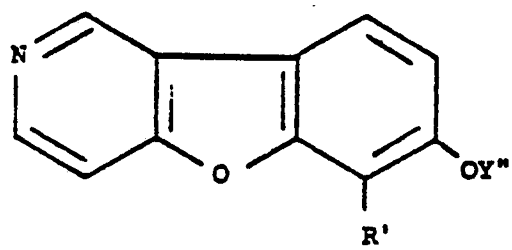

(IX)

in der Y'' für $CH_3$ steht und R' die oben genannte Bedeutung hat, d.h. für $CH_3$ oder $OCH_3$ steht;

d) man unterwirft das Zwischenprodukt IX einer Demethylierungsreaktion, um die entsprechende Verbindung der Formel IX zu liefern, in der Y'' ein Wasserstoffatom ist, nämlich ein Hydroxypyridobenzofuran;

e) man unterwirft diese letztgenannte Verbindung einer Formylierungsreaktion, um das Zwischenprodukt der Formel X

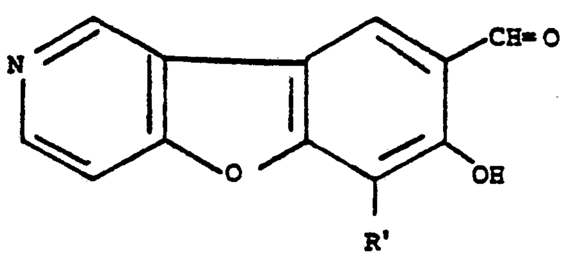

(X)

in der R' ein Methyl- oder Methoxyrest ist, zu erhalten;

f) man kondensiert den Aldehyd X mit Diethylmalonat, was zu einem Carbethoxypyrido-psoralen der Formel XI führt

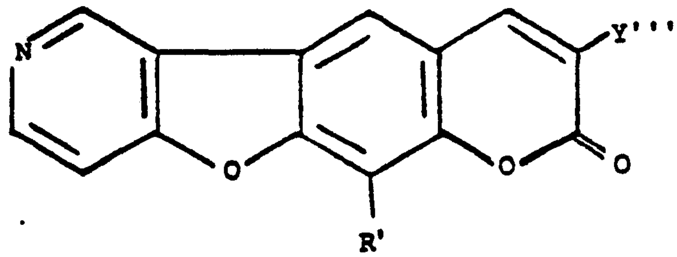

(XI)

in der Y''' der Rest $COOC_2H_5$ ist und R' = $CH_3$ oder auch $OCH_3$;
g) man hydrolysiert das Produkt XI, um die entsprechende Säure (Y''' = COOH) zu erhalten;
h) man führt die Decarboxylierung dieser Säure durch, was es ermöglicht, das Pyridopsoralen der Formel I, in der R = H und R' = $CH_3$ oder auch $OCH_3$, zu erhalten.

8. Als Zwischenprodukte für die Durchführung des Verfahrens nach Anspruch 6 die Psoralenderivate der Formel

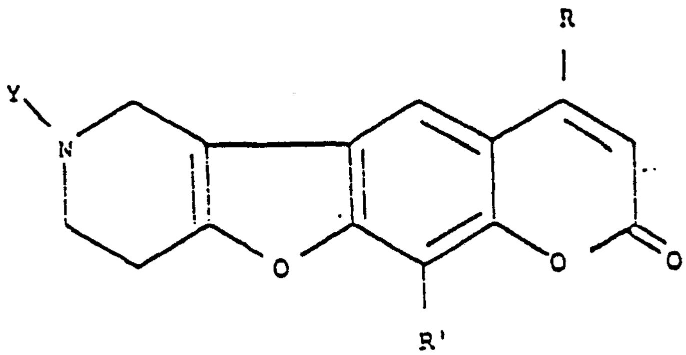

in der R und R' die in irgendeinem der Ansprüche 1 bis 5 genannte Bedeutung haben und Y ein Wasserstoffatom, ein Methylrest oder eine Benzylgruppe ist, sowie die Salze dieser Derivate, insbesondere die Hydrochloride.

9. Produkt nach Anspruch 8, worin R und R' beide ein Methylrest sind und Y ein Wasserstoffatom ist.

10. Produkt nach Anspruch 8, worin R und R' beide ein Methylrest sind und Y ein Methylrest ist.

11. Pharmazeutische Zubereitung, enthaltend als Wirkstoff wenigstens eines der Produkte nach einem der Ansprüche 1, 2, 3, 4, 5, 8, 9 und 10.

12. Pharmazeutische Zubereitung nach Anspruch 11 für die Behandlung von Hautkrankheiten, insbesondere für die Behandlung der entzündlichen, gutartigen und bösartigen Dermatosen, nämlich der Psoriasis, der fungösen Mykose, der konstitutionellen Ekzeme und Kontakt-Ekzeme, der Parapsoriasis en plaques und der Parapsoriasis guttata, der Haaransfallkrankheiten, der Prurigo-Krankheiten, der Lichen planus-Erscheinungen, der Urtika pigmentosa sowie der Pigmentstörungen und Photodermatosen.

13. Pharmazeutische Zubereitung nach einem der Ansprüche 11 oder 12, worin der Wirkstoff mit einem pharmazeutisch unbedenklichen Träger verbunden ist, für die örtliche oder orale Verabreichung.

14. Zubereitung nach Anspruch 13, dadurch gekennzeichnet, daß der Wirkstoff sich in der Zubereitung in einer Menge von 0,1 bis 2 Gew.-%, bezogen auf das Gesamtgewicht der Zubereitung, befindet.

15. Kosmetische Zusammensetzung, enthaltend als Wirkstoff wenigstens eines der Produkte nach einem der Ansprüche 1, 2, 3, 4, 5, 8, 9 und 10 oder der nach den Verfahren der Ansprüche 6 oder 7 erhaltenen Produkte, in Verbindung mit einem für die äußere Anwendung geeigneten Träger.

16. Zusammensetzung nach Anspruch 15, dadurch gekennzeichnet, daß sie eine für die Stimulierung der Pigmentierung der Haut wirksame Menge wenigstens einer der Verbindungen nach einem der Ansprüche 1 bis 5 enthält.

**Patentansprüche** für den Vertragsstaat: AT

1. Verfahren zur Herstellung von Verbindungen der Formel

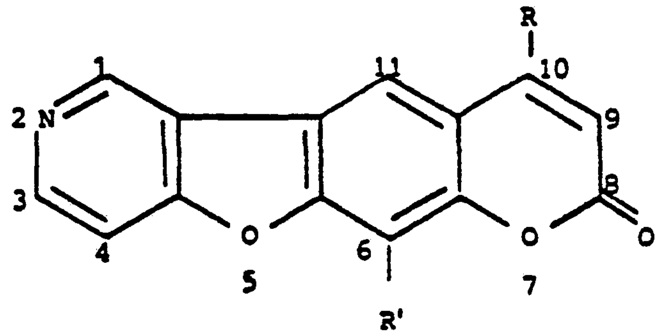

(I)

in der R ein Wasserstoffatom oder ein $C_1$-$C_4$-Alkylrest und R' ein Methyl- oder Methoxyrest ist, gekennzeichnet durch die folgenden Stufen:

a) man setzt ein 7-Aminooxy-cumarin der Formel II

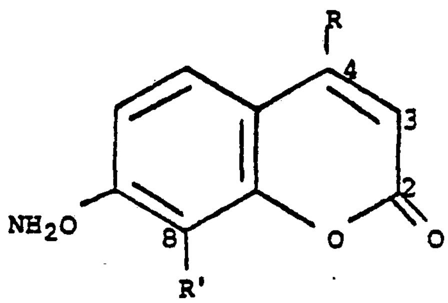

(II)

in der R und R' die oben genannte Bedeutung haben, mit einem 4-Piperidon der Formel III

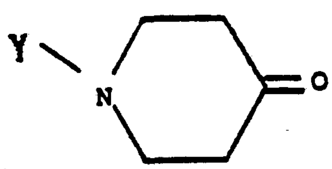

(III)

in der der Substituent Y ein Wasserstoffatom sein kann, um, was zu einem Oxim der Formel IV

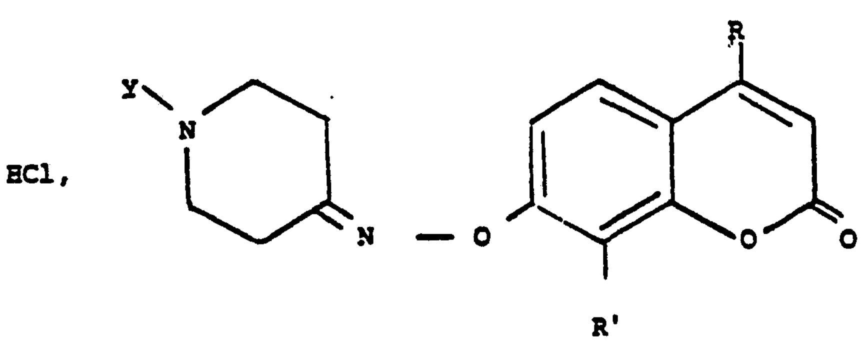

(IV)

in der R, R' und Y die oben genannten Bedeutungen haben, führt.

b) man führt die Cyclisierung des Oxims IV in saurem Medium durch, was zu einem 1,2,3,4-Tetrahydropyrido[3',4':4,5]psoralen der Formel V

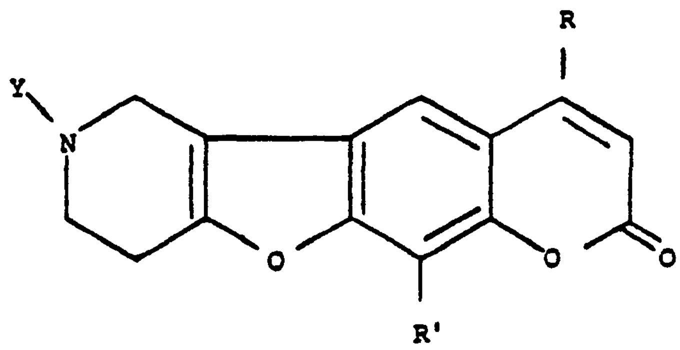

(V)

in der R, R' und Y die oben genannte Bedeutung haben, führt;

c) man führt die Aromatisierung des Produkts V in Gegenwart eines Dehydriermittels durch, um schließlich die gewünschte Verbindung der Formel I zu erhalten.

2. Verfahren zur Herstellung der Verbindungen, die der in Anspruch 1 genannten Formel I entsprechen, in der R ein Wasserstoffatom und R' ein Methyl- oder Methoxyrest ist, gekennzeichnet durch die folgenden Stufen:

a) man setzt Monomethylether von 2-Methyl- oder 2-Methoxyresorcin der Formel VI

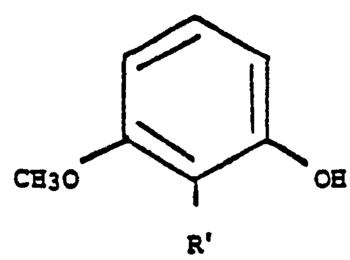

(VI)

in der R' = $CH_3$ oder $OCH_3$, mit 3-Nitro-4-chlorpyridin der Formel VII

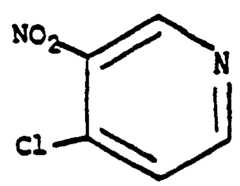

(VII)

um, was zu einem nitrierten Zwischenderivat entsprechend der Formel VIII

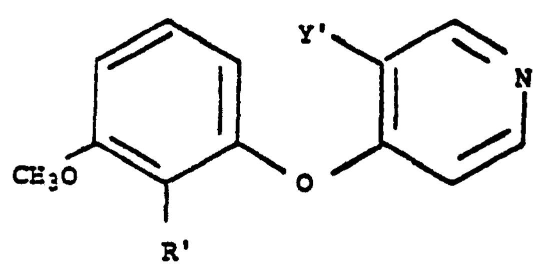

(VIII)

in der R' für $CH_3$ oder $OCH_3$ steht und Y' = $NO_2$, führt;

b) man wandelt das nitrierte Produkt (VIII, worin Y' = $NO_2$) in das entsprechende Amin um, in dem Y' = $NH_2$, und diazotiert anschließend dieses Amin, um das entsprechende Produkt mit Y' = $N_2^+Cl^-$ zu liefern;

c) man cyclisiert dieses letztgenannte Produkt, um die Verbindung der Formel IX

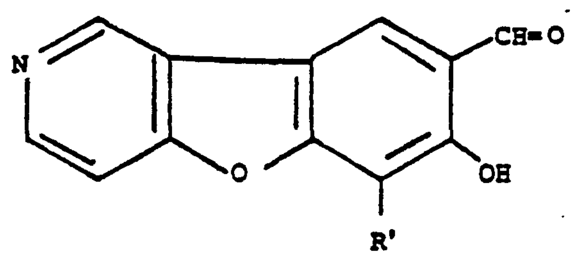

(X)

zu erhalten, in der Y'' für $CH_3$ steht und R' die oben genannte Bedeutung, d.h. $CH_3$ oder $OCH_3$, hat;

d) man unterwirft das Zwischenprodukt IX einer Demethylierungsreaktion, um die entsprechende Verbindung der Formel IX zu liefern, in der Y'' ein Wasserstoffatom ist;

e) man unterwirft diese letztgenannte Verbindung einer Formylierungsreaktion, um das Zwischenprodukt der Formel X

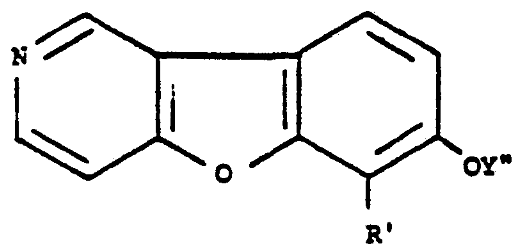

(IX)

in der R' ein Methyl- oder Methoxyrest ist, zu erhalten;

f) man kondensiert den Aldehyd X mit Diethylmalonat, was zu einer Verbindung der Formel XI

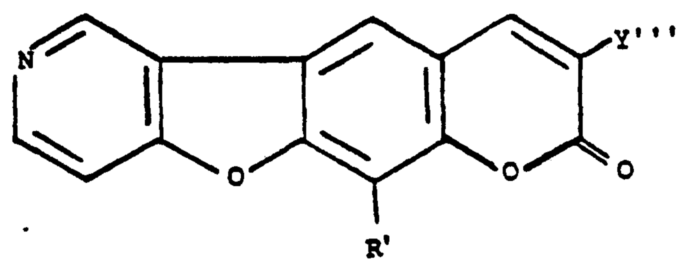

(XI)

in der Y''' den Rest $COOC_2H_5$ darstellt und R' = $CH_3$ oder auch $OCH_3$, führt;

g) man hydrolysiert das Produkt XI, um die entsprechende Säure (Y''' = COOH) zu erhalten;

h) man führt die Decarboxylierung dieser Säure durch, was es ermöglicht, die Verbindung der Formel I, in der R = H und R' = $CH_3$ oder auch $OCH_3$, zu erhalten.

3. Verfahren nach einem der Ansprüche 1 oder 2 zur Herstellung einer Verbindung, in der R für H steht und R' $CH_3$ ist.

4. Verfahren nach Anspruch 1 zur Herstellung einer Verbindung, in der R und R' für $CH_3$ stehen.

5. Verfahren zur Herstellung einer pharmazeutischen Zubereitung, dadurch gekennzeichnet, daß man mit einem pharmazeutisch unbedenklichen Hilfsstoff eine therapeutisch wirksame Menge einer Verbindung mischt, die der vorstehenden Formel I entspricht, in der R und R' die in Anspruch 1 genannten Bedeutungen haben.

6. Verfahren nach Anspruch 5, in dem R für H oder $CH_3$ und R' für $CH_3$ stehen.

7. Verfahren nach einem der Ansprüche 5 oder 6, dadurch gekennzeichnet, daß der Wirkstoff in einer Menge von 0,1 bis 2 Gew.-% der Zubereitung vorhanden ist.

8. Verfahren zur Herstellung einer kosmetischen Zubereitung für die örtliche Anwendung, dadurch gekennzeichnet, daß man mit einem qeeigneten Träger eine Verbindung entsprechend der vorstehenden Formel I mischt, in der R und R' die in Anspruch 1 genannten Bedeutungen haben.

9. Verfahren nach Anspruch 8, in dem R für H oder $CH_3$ und R' für $CH_3$ stehen.

**Claims** for the contracting States

BE CH DE GB IT LI LU NL SE

1. Pyrido[3,4-h]psoralens or pyrido[3',4':4,5]furo-[3,2-g]coumarins corresponding to the formula I

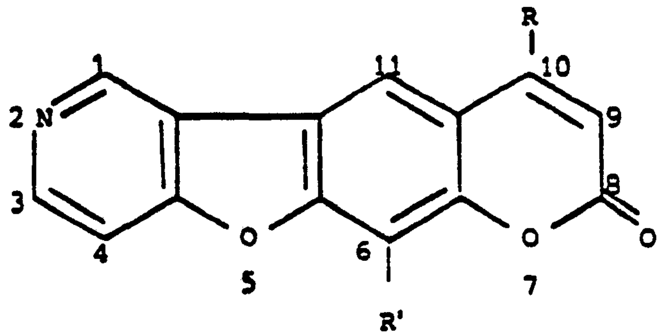

(I)

in which formula R is a hydrogen atom or a $C_1$-$C_4$ alkyl group and R' is a methyl or methoxy group.

2. Compounds according to Claim 1, in which R is a methyl group or a hydrogen atom.

3. Compounds according to one of Claims 1 and 2 in which R' is a methyl group.

4. Compound according to Claim 1, in which R is a hydrogen atom and R' is a methyl group.

5. Compound according to Claim 1, in which R and R' both denote a methyl group.

6. Process for obtaining the compounds according to any one of Claims 1 to 5, characterized by the following stages:

(a) a 7-(aminooxy)coumarin of formula II

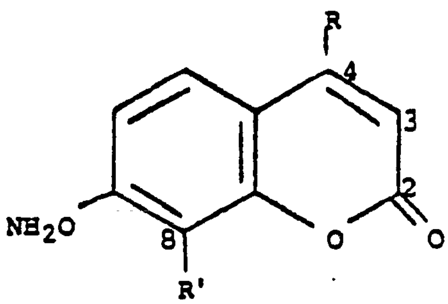

(II)

in which R and R' have the meaning stated above, is reacted with a 4-piperidone of formula III

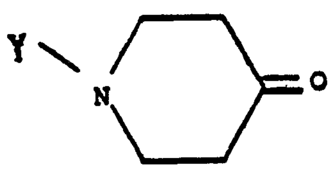

(III)

in which the substituent Y can be a hydrogen atom, which leads to an oxime of formula IV

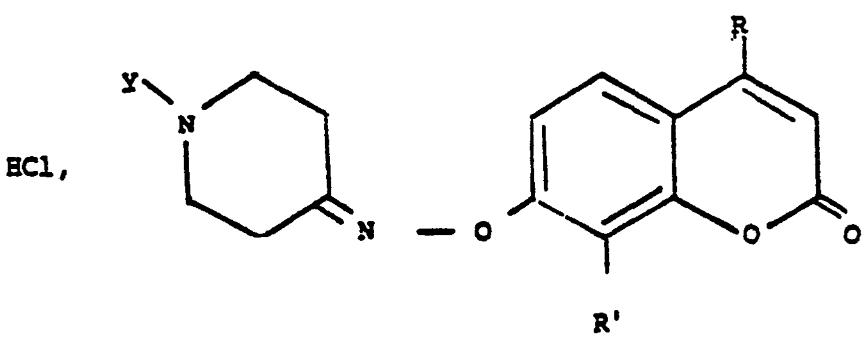

(IV)

in which R, R' and Y have the meanings stated above;

(b) cyclization of the oxime IV is carried out in acid medium, which leads to a 1,2,3,4-tetrahydropyrido-[3',4':4,5]psoralen of formula V

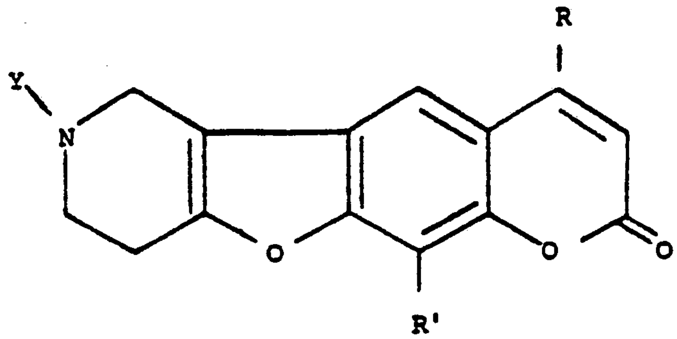

(V)

in which R, R' and Y have the meaning stated above;

(c) aromatization of the product V is performed in the presence of a dehydrogenating agent, to obtain finally the desired compound of formula I.

7. Process for obtaining the compounds according to any one of Claims 1 to 4, in which R is a hydrogen atom and R' a methyl or methoxy radical, said process being characterized by the following stages:

(a) 2-methyl- or 2-methoxyresorcinol monomethyl ether-of formula VI

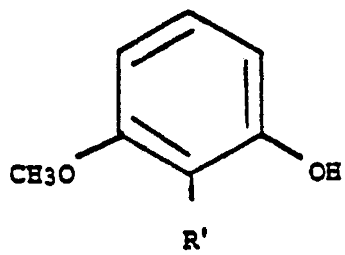

(VI)

in which R' = $CH_3$ or $OCH_3$, is reacted with 3-nitro-4-chloropyridine of formula VII

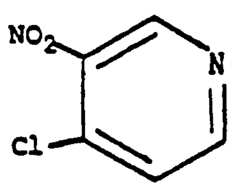

(VII)

which leads to an intermediate nitro derivative corresponding to the formula VIII

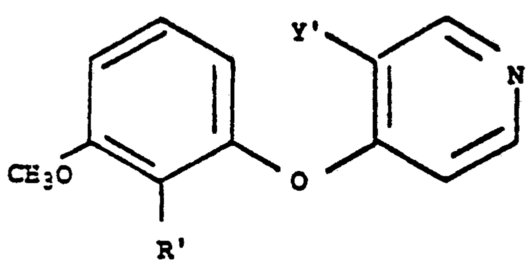

(VIII)

in which R' is $CH_3$ or $OCH_3$ and Y' = $NO_2$;

(b) the nitro product (VIII in which Y' = $NO_2$) is converted to the corresponding amine in which Y' = $NH_2$, which is then diazotized to yield the corresponding product with Y' = $N_2^+Cl^-$;

(c) the latter product is cyclized to obtain the compound of formula IX

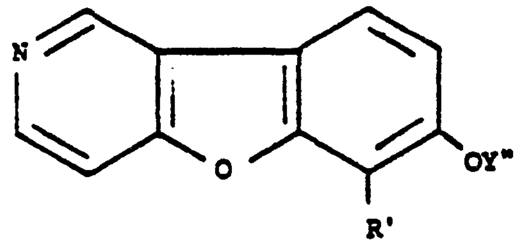

(IX)

in which Y" denotes $CH_3$ and R' has the meaning stated above, namely $CH_3$ or $OCH_3$;

(d) the intermediate product IX is subjected to a demethylation reaction to yield the corresponding compound of formula IX in which Y" is a hydrogen atom, namely a hydroxypyridobenzofuran;

(e) the latter compound is subjected to a formylation reaction to obtain the intermediate product of formula X

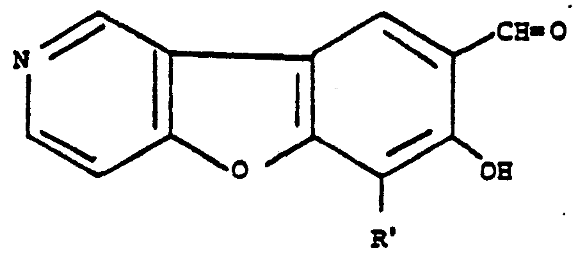

(X)

in which R' denotes a methyl or methoxy radical;

(f) the aldehyde X is condensed with diethyl malonate, which leads to a carbethoxypyridopsoralen of formula XI

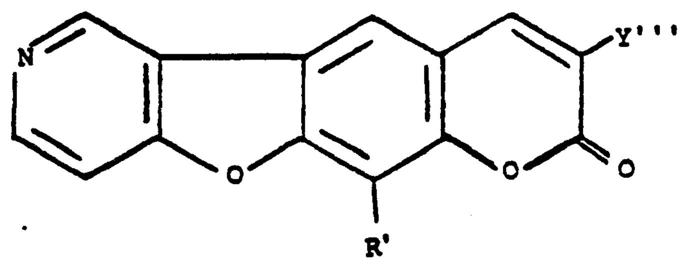

(XI)

in which Y''' denotes a $COOC_2H_5$ radical and R' = $CH_3$ or alternatively $OCH_3$;

(g) the product XI is hydrolysed to obtain the corresponding acid (Y''' = COOH);

(h) decarboxylation of this acid is performed, which enables the pyridopsoralen of formula I in which R = H and R' = $CH_3$ or alternatively $OCH_3$ to be obtained.

8. By way of intermediate products for carrying outthe process according to Claim 6, psoralen derivatives of formula

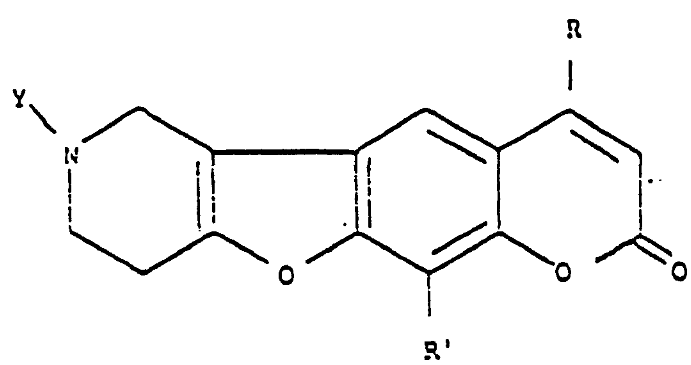

in which R and R' have the meaning stated in any one of Claims 1 to 5 and Y is a hydrogen atom, a methyl radical or a benzyl group, as well as the salts of these derivatives, especially the hydrochlorides.

9. Product according to Claim 8, in which R and R' both denote a methyl radical and Y is a hydrogen atom.

10. Product according to Claim 8, in which R and R′ both denote a methyl radical and Y is a methyl group.

11. Pharmaceutical composition containing, by way of an active agent, at least one of the products according to one of Claims 1, 2, 3, 4, 5, 8, 9 and 10.

12. Pharmaceutical composition according to Claim 11, for the treatment of skin conditions and more especially for the treatment of benign and malignant inflammatory dermatoses, namely psoriasis, mycosis fungoides, constitutional and contact eczemas, parapsoriasis en plaques and parapsoriasis guttata, alopecia, prurigos, lichen planus and urticaria pigmentosa, as well as disorders of pigmentation and photodermatoses.

13. Pharmaceutical composition according to one of Claims 11 and 12, in which the active agent is combined with a pharmaceutically acceptable vehicle for local or oral administration.

14. Composition according to Claim 13, characterized in that the active agent is present in the composition in the proportion of 0.1 to 2% by weight, relative to the total weight of the composition.

15. Cosmetic composition containing, by way of an active agent, at least one of the products according to one of Claims 1, 2, 3, 4, 5, 8, 9 and 10, or obtained by the processes of Claim 6 or 7, in combination with a vehicle suitable for external application.

16. Composition according to Claim 15, characterized in that it contains an amount which is effective for stimulating the pigmentation of the skin, of at least one of the compounds according to one of Claims 1 to 5.

**Claims** for the contracting State AT

1. Process for obtaining the compounds of formula

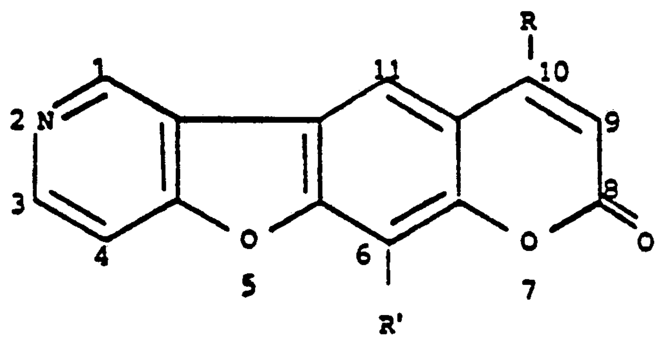

(I)

in which R is a hydrogen atom or a $C_1$-$C_4$ alkyl group and R′ is a methyl or methoxy group, characterized by the following stages:

(a) a 7-(aminooxy)coumarin of formula II

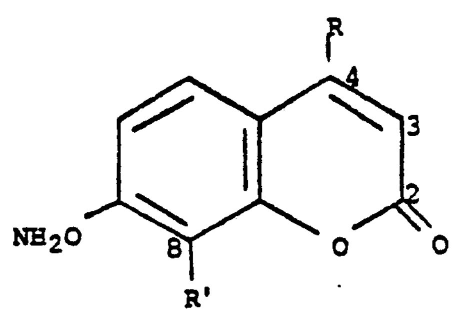

(II)

in which R and R′ have the meaning stated above, is reacted with a 4-piperidone of formula III

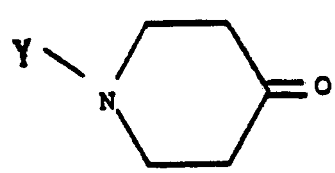

(III)

in which the substituent Y can be a hydrogen atom, which leads to an oxime of formula IV

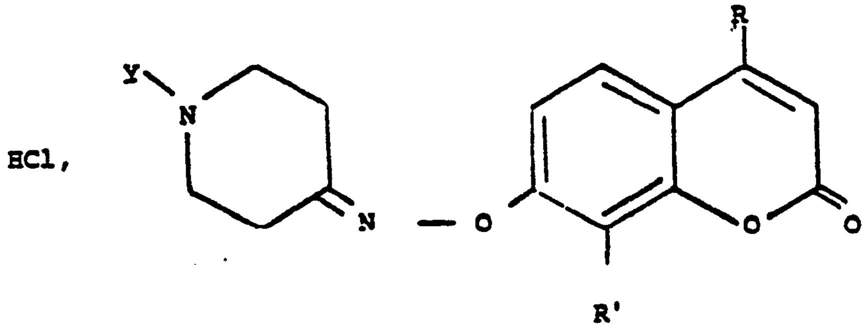

(IV)

in which R, R′ and Y have the meanings stated above;

(b) cyclization of the oxime IV is carried out in acid medium, which leads to a 1,2,3,4-tetrahydropyrido-[3′,4′:4,5]psoralen of formula V

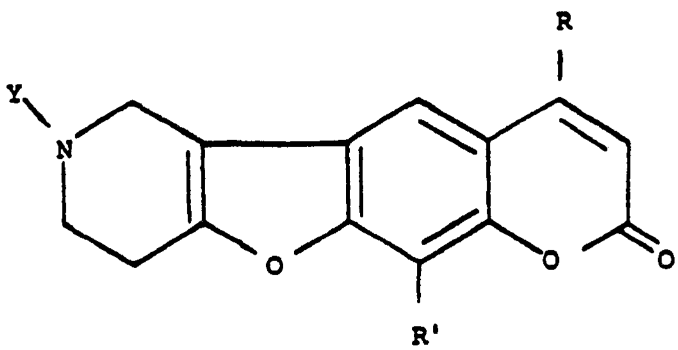

(V)

in which R, R′ and Y have the meaning stated above;

(c) aromatization of the product V is performed in the presence of a dehydrogenating agent, to obtain finally the desired compound of formula I.

2. Process for obtaining the compounds corresponding to formula I shown in Claim 1, in which R is a hydrogen atom and R′ a methyl or methoxy radical, characterized by the following stages:

(a) 2-methyl- or 2-methoxyresorcinol monomethyl ether of formula VI

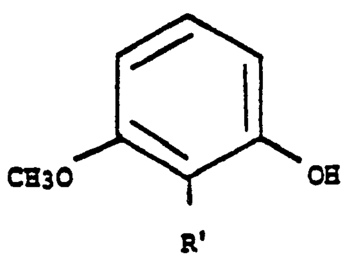

(VI)

in which R′ = $CH_3$ or $OCH_3$, is reacted with 3-nitro-4-chloropyridine of formula VII

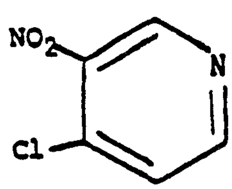

(VII)

which leads to an intermediate nitro derivative corresponding to the formula VIII

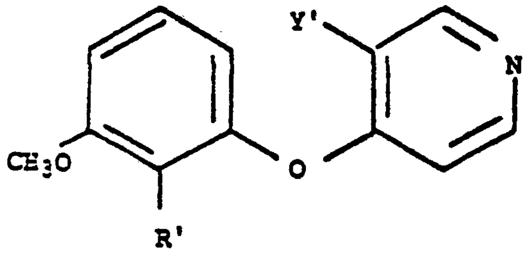

(VIII)

in which R′ is $CH_3$ or $OCH_3$ and Y′ = $NO_2$;

(b) the nitro product (VIII in which Y′ = $NO_2$) is converted to the corresponding amine in which Y′ = $NH_2$, which is then diazotized to yield the corresponding product with Y′ = $N_2^+Cl^-$;

(c) the latter product is cyclized to obtain the compound of formula IX

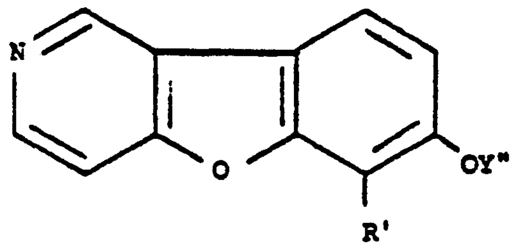

(IX)

in which Y″ denotes $CH_3$ and R′ has the meaning stated above, namely $CH_3$ or $OCH_3$;

(d) the intermediate product IX is subjected to a demethylation reaction to yield the corresponding compound of formula IX in which Y″ is a hydrogen atom;

(e) the latter compound is subjected to a formylation reaction to obtain the intermediate product of formula X

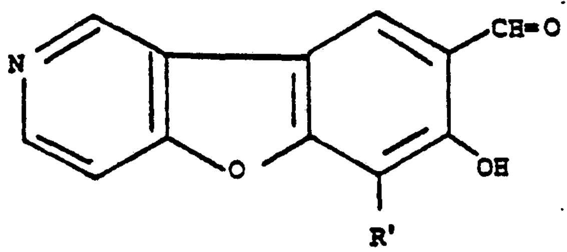

(X)

in which R′ denotes a methyl or methoxy radical;

(f) the aldehyde X is condensed with diethyl malonate, which lead to a compound of formula XI

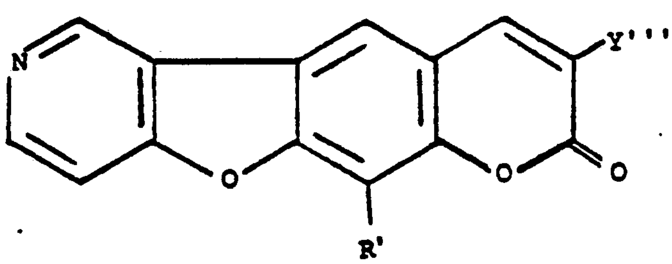

(XI)

in which Y‴ denotes a $COOC_2H_5$ radical and R′ = $CH_3$ or alternatively $OCH_3$;

(g) the product XI is hydrolysed to obtain the corresponding acid (Y‴ = COOH);

(h) decarboxylation of this acid is performed, which enables the compound of formula I in which R = H and R′ = $CH_3$ or alternatively $OCH_3$ to be obtained.

3. Process according to one of Claims 1 and 2, for obtaining a compound in which R is H and R′ is $CH_3$.

4. Process according to Claim 1, for obtaining a compound in which R and R' denote $CH_3$.

5. Process for preparing a pharmaceutical composition, characterized in that a therapeutically effective amount of a compound corresponding to the formula I above, in which R and R' have the meanings given in Claim 1, is mixed with a pharmaceutically acceptable excipient.

6. Process according to Claim 5 in which R is H or $CH_3$ and R' is $CH_3$.

7. Process according to one of Claims 5 and 6, characterized in that the active agent is present in the proportion of 0.1 to 2% by weight of the composition.

8. Process for preparing a cosmetic composition for local application, characterized in that a compound corresponding to the formula I above, in which R and R' have the meanings given in Claim 1, is mixed with a suitable vehicle.

9. Process according to Claim 8, for which R is H or $CH_3$ and R' is $CH_3$.

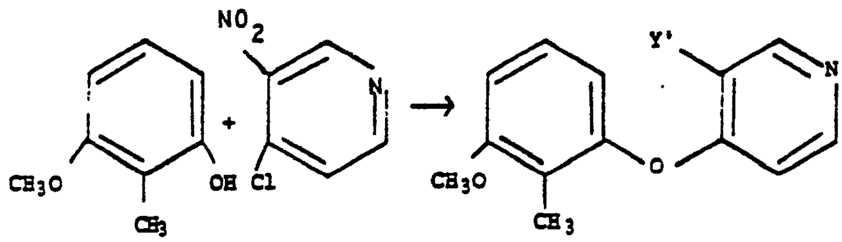

Composé 6 Composé 7 Composés 8a $Y'=NO_2$

8b $Y'=NH_2$

8c $Y'=N_2^+Cl^-$

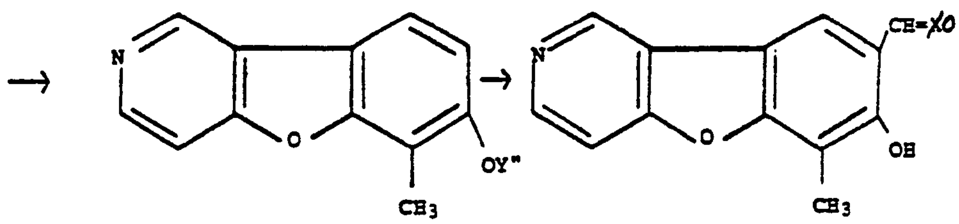

composés 9a $Y''=CH_3$ Composé 10a

9b $Y''=H$

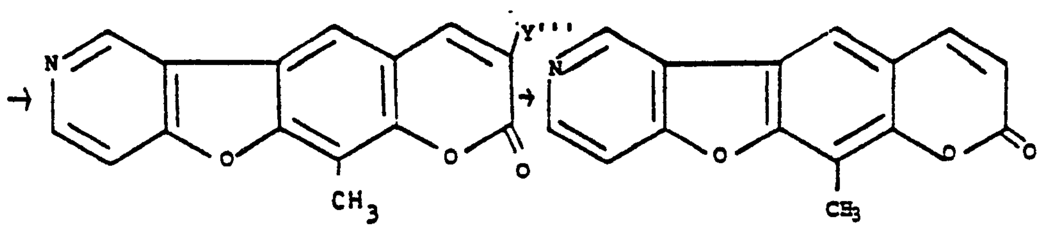

Composés 11a $Y'''=COOC_2H_5$

11b $Y'''=COOH$